(19) Europäisches Patentamt
European Patent Office
Office européen des brevets

(11) EP 4 783 193 A1

(12) EUROPEAN PATENT APPLICATION

(43) Date of publication:
29.07.2026 Bulletin 2026/31

(21) Application number: 26153915.9

(22) Date of filing: 23.01.2026

(51) International Patent Classification (IPC):
G16H 50/20 (2018.01)   G06T 7/00 (2017.01)
G16H 50/30 (2018.01)   G16H 30/40 (2018.01)

(52) Cooperative Patent Classification (CPC):
G16H 50/20; G06T 7/0012; G16H 50/30;
G06T 2207/10132; G06T 2207/20084; G16H 30/40

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 24.01.2025 US 202563749425 P

(71) Applicant: Deep Breathe Inc.
London, ON N6A 1B8 (CA)

(72) Inventors:
• Vanberlo, Blake
Lucan, N0M 2J0 (CA)

• Prager, Ross Thomas
London, N6C 1C8 (CA)
• Pace, Jacob
London, L9L 1B4 (CA)
• Smith, Delaney
Port Perry, L9L 1B4 (CA)
• Arntfield, Robert Thomas
London, N6C 2G5 (CA)
• Bartman, Daniel
Mississauga, L5L 0A9 (CA)
• Rahman, Marwan
Mississauga, L5B 1J9 (CA)

(74) Representative: Marks & Clerk LLP
15 Fetter Lane
London EC4A 1BW (GB)

(54) **COMPUTING SYSTEMS AND METHODS FOR AUTOMATICALLY PROCESSING TORSO ULTRASOUND**

(57) Computing systems and methods for detecting a pathological condition based on a set of one or more ultrasound image of a torso. The method includes: processing the set of one or more ultrasound image of the torso using one or more neural networks to generate: a prediction of a presence and/or position of each of a plurality of anatomical structures in the set of one or more ultrasound image of the torso, and a prediction of a presence of the pathological condition and/or a quantitative indication of a severity of the pathological condition; and in response to determining, based on the prediction of the presence and/or position of the plurality of anatomical structures, that the set of one or more ultrasound image is suitable for detecting the pathological condition, outputting the prediction of the presence of the pathological condition and/or the quantitative indication of the severity of the pathological condition.

FIG. 1

EP 4 783 193 A1

**Description**

**TECHNICAL FIELD**

The disclosed exemplary embodiments relate to computer-implemented systems and methods for automatically processing torso ultrasound.

**BACKGROUND**

Blunt force trauma patients often have injuries that are difficult to identify through an initial physical exam. This is particularly true in relation to blunt force trauma to the abdomen (i.e., blunt abdominal trauma (BAT)) where, as the result of, for example, a spleen or liver injury, intraperitoneal bleeding occurs.

While a CT (computed tomography) scan is the gold standard for diagnosing intra-abdominal injuries, such as intraperitoneal bleeding. Time delays and transportation away from the initial diagnosis site (e.g., emergency room) to obtain a CT scan, makes it difficult to rely on a CT scan for a quick diagnosis, particular with unstable patients. Accordingly, ultrasound, with its many advantages, including, but not limited to, bedside availability, ease of use, and reproducibility has become the standard for rapid diagnosis of intraperitoneal bleeding (i.e., bleeding within the peritoneal cavity which is the space that contains the abdominal organs) after blunt force abdominal trauma.

Specifically, an ultrasound protocol, referred to as Focused Assessment with Sonography in Trauma (FAST) examination, has been developed to detect intraperitoneal bleeding, and more particularly hemoperitoneum (i.e., a condition that occurs when blood accumulates in the peritoneal cavity) and hemopericardium (i.e., a condition that occurs when blood accumulates in the sac around the heart). The FAST examination evaluates spaces where free fluid could accumulate - the pericardium, right upper quadrant (RUQ), left upper quadrant (LUQ) and pelvic region. More particularly, an ultrasound technician obtains, one at a time, ultrasound images representing four different views of the patient's torso - a RUQ view, a LUQ view, a pelvic (or suprapubic) view and a subxiphoid view. The ultrasound technician or another person trained to interpret ultrasound images analyzes the obtained images to determine if there is free fluid in the pericardium, right upper quadrant (RUQ), left upper quadrant (LUQ) and/or pelvic region indicating traumatic injury. On ultrasound, free fluid generally appears anechoic. Full guidelines for FAST examination have been published by the American Institute of Ultrasound in Medicine (AIUM) and the American College of Emergency Physicians (ACEP).

**SUMMARY**

The following summary is intended to introduce the reader to various aspects of the detailed description, but not to define or delimit any invention.

A first aspect provides a system to detect a pathological condition based on a set of one or more ultrasound image of a torso, the system comprising: a memory storing instructions; and at least one processor coupled to the memory, the at least one processor configured to execute the instructions to perform a method comprising: processing the set of one or more ultrasound image of the torso using one or more neural networks to generate: a prediction of a presence and/or position of each of a plurality of anatomical structures in the set of one or more ultrasound image of the torso, and a prediction of a presence of the pathological condition and/or a quantitative indication of a severity of the pathological condition; and in response to determining, based on the prediction of the presence and/or position of the plurality of anatomical structures, that the set of one or more ultrasound image is suitable for detecting the pathological condition, outputting the prediction of the presence of the pathological condition and/or the quantitative indication of the severity of the pathological condition.

The one or more neural networks may comprise a single neural network configured to generate the prediction of the presence and/or position of each of the plurality of anatomical structures in the set of one or more ultrasound image of the torso, and the prediction of the presence of the pathological condition and/or the quantitative indication of the severity of the pathological condition.

The one or more neural networks may comprise a first neural network configured to generate the prediction of the presence and/or position of each of the plurality of anatomical structures in the set of one or more ultrasound image of the torso and a second, different neural network configured to generate the prediction of the presence of the pathological condition and/or the quantitative indication of the severity of the pathological condition.

The method may further comprise only processing the set of one or more ultrasound image of the torso using the second, different, neural network in response to determining that the set of one or more ultrasound image of the torso is suitable for detecting the pathological condition.

The second, different neural network may be configured to process the set of one or more ultrasound image of the torso in conjunction with data generated from the prediction of the presence and/or position of each of the plurality of anatomical structures in the set of one or more ultrasound image of the torso.

The one or more neural networks may comprise a classifier neural network configured to generate the prediction of the presence of each of the plurality of anatomical structures in the set of one or more ultrasound image of the torso and the prediction of the presence of each of the plurality of anatomical structures comprises a multi-element vector that comprises an element for each of the plurality of anatomical structures that indicates the prediction of the presence of that anatomical structure in the set of one or more ultrasound image of the torso.

Determining, based on the prediction of the presence and/or position of the plurality of anatomical structures, whether the set of one or more ultrasound image of the torso is suitable for detecting the pathological condition may comprise: generating a binary decision as to whether the set of one or more ultrasound image of the torso is suitable for detecting the pathological condition; or generating a ternary decision as to whether the set of one or more ultrasound image of the torso is suitable for detecting the pathological condition, the ternary decision indicating whether the set of one or more ultrasound image of the torso is (i) suitable for detecting the pathological condition, (ii) not suitable for detecting the pathological condition, or (iii) comprises an anatomically impossible combination of the plurality of anatomical structures.

The set of one or more ultrasound image of the torso may comprise a plurality of ultrasound images of the torso and processing the set of one or more ultrasound image of the torso using the one or more neural networks to generate the prediction of the presence and/or position of each of the plurality of anatomical structures in the set of one or more ultrasound image may comprise: processing the plurality of ultrasound images of the torso as a whole using a neural network of the one or more neural networks to generate the prediction of the presence and/or position of each of the plurality of anatomical structures; or processing each ultrasound image of the plurality of ultrasound images of the torso using a neural network of the one or more neural networks to generate a prediction of the presence and/or position of each of the plurality of anatomical structures in that ultrasound image, and generating the prediction of the presence and/or position of each of the plurality of anatomical structures in the set of one or more ultrasound image of the torso based on the prediction of the presence and/or position of that anatomical structure for each ultrasound image of the plurality of ultrasound images of the torso.

The one or more neural networks may comprise an object detection neural network configured to generate the prediction of the position of each of the plurality of anatomical structures in the set of one or more ultrasound image of the torso.

The one or more neural networks may comprise a segmentation neural network configured to generate the prediction of the position of each of the plurality of anatomical structure in the set of one or more ultrasound image of the torso.

The one or more neural networks may comprise a classifier neural network configured to generate the prediction of the presence of the pathological condition, and the prediction of the presence of the pathological condition may comprise (i) a single value that indicates whether the pathological condition is predicted to be present; or (ii) a plurality of values that indicate varying degrees of detection of the pathological condition.

The set of one or more ultrasound image of the torso may comprise a plurality of ultrasound images of the torso and a neural network of the one or more neural networks is configured to: process the plurality of ultrasound images of the torso as a whole to generate the prediction of the presence of the pathological condition and/or the quantitative indication of the severity of the pathological condition; or process each ultrasound image of the plurality of ultrasound images of the torso separately to generate a prediction of the presence of the pathological condition and/or the quantitative indication of the severity of the pathological condition for that ultrasound image, and generate the prediction of the presence of the pathological condition and/or the quantitative indication of the severity of the pathological condition based on the prediction of the presence of the pathological condition and/or the quantitative indication of the severity of the pathological condition for each ultrasound image of the plurality of ultrasound images of the torso.

The one or more neural networks may comprise a neural network configured to generate the quantitative indication of the severity of the pathological condition, the quantitative indication of the severity of the pathological condition comprising, for one or more ultrasound image in the set of one or more ultrasound image of the torso, a segmentation mask that indicates which pixels of the ultrasound image correspond to the pathological condition.

The one or more neural networks may comprise a neural network that is configured to generate the quantitative indication of the severity of the pathological condition and that neural network comprises: an object detection neural network configured to identify bounding boxes of continuous segments in the set of one or more ultrasound image of the torso that correspond to the pathological condition; and a model configured to generate a segmentation mask for each identified bounding box that indicates which pixels of that bounding box correspond to the pathological condition.

The one or more neural networks may comprise a neural network that is configured to generate the quantitative indication of the severity of the pathological condition and that neural network may comprise: a neural network configured to generate one or more segmentation mask from the one or more ultrasound image of the torso; and a model configured to determine an area of the set of one or more ultrasound image of the torso corresponding to the pathological condition based on the one or more segmentation mask.

Each segmentation mask may be confined to pixels of a corresponding ultrasound image corresponding to an ultrasound beam.

The one or more neural networks may comprise a features extractor configured to generate the quantitative indication of the severity of the pathological condition, wherein the quantitative indication of the severity of the pathological condition comprises a value that represents a predicted severity of the pathological condition.

The pathological condition may be free fluid in the torso.

The quantitative indication of the severity of the pathological condition may be an estimate of a quantity of the free fluid in the torso.

A second aspect provides a method for detecting a pathological condition based on a set of one or more ultrasound image of a torso, the method comprising, at one or more processors: processing the set of one or more ultrasound image of the torso using one or more neural networks to generate: a prediction of a presence and/or position of each of a plurality of anatomical structures in the set of one or more ultrasound image; and a prediction of presence of the pathological condition and/or a quantitative indication of a severity of the pathological condition; and in response to determining, based on the prediction of the presence and/or position of the plurality of anatomical structures, that the set of one or more ultrasound image of the torso is suitable for detecting the pathological condition, outputting the prediction of the presence of the pathological condition and/or the quantitative indication of the severity of the pathological condition.

A third aspect provides a method of processing data to detect a pathological condition based on a set of one or more ultrasound image of a torso, the method comprising: (a) processing the set of one or more ultrasound image using a first neural network to generate a prediction of presence and/or position of each of a plurality of anatomical structures in the set of one or more ultrasound image; (b) determining whether the set of one or more ultrasound image is suitable for detecting the pathological condition based on the predicted presence of the plurality of anatomical structures; and (c) in response to determining that the set of one or more ultrasound image is suitable for detecting the pathological condition, processing the set of one or more ultrasound image using a second neural network to generate a prediction of presence of the pathological condition and/or a quantitative indication of a severity of the pathological condition.

The set of one or more ultrasound image may be processed using the first neural network to generate the prediction of the presence of each of a plurality of anatomical structures in the set of one or more ultrasound image and the prediction of the presence of each of the plurality of anatomical structures may comprise a multi-element vector in which each element of the multi-element vector comprises the prediction of the presence of one of the plurality of anatomical structures in the set of one or more ultrasound image.

The method may further comprise, prior to determining whether the set of one or more ultrasound image is suitable for detecting the pathological condition, binarizing the multi-element vector by applying, to each element of the multi-element vector, a corresponding threshold.

The first neural network may comprise a multi-label image classifier neural network that is configured to, in response to receiving one or more ultrasound images of the torso, generate a multi-element vector in which each element of the multi-element vector comprises the prediction of the presence of one of the plurality of anatomical structures in the received one or more ultrasound images.

A final layer of the first neural network may comprise a fully connected layer followed by an activation function, and the final layer is configured to generate the multi-element vector.

The activation function may be a sigmoid function.

The multi-label image classifier neural network may be a convolutional neural network or a vision transformer neural network.

Determining whether the set of one or more ultrasound image is suitable for detecting the pathological condition based on the predicted presence and/or position of the plurality of anatomical structures may comprise generating a binary decision as to whether the set of one or more ultrasound image is suitable for detecting the pathological condition.

Determining whether the set of one or more ultrasound image is suitable for detecting the pathological condition based on the predicted presence and/or position of the plurality of anatomical structures may comprise generating a ternary decision as to whether the set of one or more ultrasound image is suitable for detecting the pathological condition, the ternary decision indicating whether the set of one or more ultrasound image is suitable for detecting the pathological condition, not suitable for detecting the pathological condition, or comprises an anatomically impossible combination of the plurality of anatomical structures.

The set of one or more ultrasound image may comprise a plurality of ultrasound images and processing the set of one or more ultrasound image using the first neural network to generate the prediction of the presence and/or position of each of the plurality of anatomical structures in the set of one or more ultrasound image may comprise processing the plurality of images as a whole using the first neural network to generate the prediction of the presence and/or position of each of the plurality of anatomical structures

The set of one or more ultrasound image may comprise a plurality of ultrasound images and processing the set of one or more ultrasound image using the first neural network to generate the prediction of the presence and/or position of each of the plurality of anatomical structures in the set of one or more ultrasound image may comprise: processing each ultrasound image of the plurality of ultrasound images using the first neural network to generate a prediction of the presence and/or position of each of the plurality of anatomical structures in that ultrasound image; and generating the prediction of the presence and/or position of each of the plurality of anatomical structures in the set of one or more ultrasound image based on the prediction of the presence and/or position of each of the plurality of anatomical structures for each ultrasound image of the plurality of ultrasound images.

Generating the prediction of the presence of each of the plurality of anatomical structures in the set of one or more ultrasound image based on the prediction of the presence of each of the plurality of anatomical structures for each ultrasound image of the plurality of ultrasound images may comprise computing, for each anatomical structure of the plurality of anatomical structures, a mean of the predictions for that anatomical structure.

Generating the prediction of the presence of each of the plurality of anatomical structures in the set of one or more ultrasound image based on the prediction of the presence of each of the plurality of anatomical structures for each ultrasound image of the plurality of ultrasound images may comprise: applying a moving average with a predetermined window size to the prediction for each anatomical structure of the plurality of structures; and predicting that an anatomical structure is present in the set of one or more ultrasound image if there are a predetermined number of ultrasound images wherein the prediction for that anatomical structure exceeds a threshold.

The set of one or more ultrasound image may be processed using the first neural network to generate the prediction of the position of each of a plurality of anatomical structures in the set of one or more ultrasound image and the first neural network may comprise an object detection neural network.

The first neural network may comprise an instance segmentation neural network.

The set of one or more ultrasound image may be processed using the second neural network to generate the prediction of the presence of the pathological condition and the second neural network may be a classifier neural network.

The classifier neural network may be a single class classifier neural network and the prediction of the presence of the pathological condition may comprise a single value that indicates whether the pathological condition is predicted to be present.

The classifier neural network may comprise a multi-class classifier neural network and the prediction of the presence of the pathological condition may comprise a plurality of values that indicate varying degrees of detection of the pathological condition.

The multi-class classifier neural network may be configured to classify the set of one or more ultrasound image as (1) the pathologic condition is present, (2) the pathologic condition is possibly present, or (3) the pathological condition is not present.

The plurality of values may comprise a value for each class, and the method may further comprise applying a SoftMax activation function to the plurality of values.

The number of values in the plurality of values may be less than the number of classes detected by the multi-class classifier neural network.

The set of one or more ultrasound image may comprise a plurality of ultrasound images and processing the set of one or more ultrasound image using the second neural network to generate the prediction of the presence of the pathological condition and/or the quantitative indication of a severity of the pathological condition may comprise processing the plurality of ultrasound images as a whole using the second neural network to generate the prediction of the presence of the pathological condition and/or the quantitative indication of a severity of the pathological condition.

The set of one or more ultrasound image may comprise a plurality of ultrasound images and processing the set of one or more ultrasound image using the second neural network to generate the prediction of the presence of the pathological condition and/or the quantitative indication of a severity of the pathological condition may comprise: processing each ultrasound image of the plurality of ultrasound images using the second neural network to generate a prediction of the presence of the pathological condition and/or a quantitative indication of a severity of the pathological condition for that ultrasound image; and generating the prediction of the presence of the pathological condition and/or the quantitative indication of a severity of the pathological condition based on the prediction of the presence of the pathological condition and/or the quantitative indication of a severity of the pathological condition for each ultrasound image of the plurality of ultrasound images.

The set of one or more ultrasound image may be processed using the second neural network to generate the quantitative indication of the severity of the pathological condition and the second neural network may comprise a deep neural network that is configured to receive an ultrasound image and generate a segmentation mask that indicates which pixels of the ultrasound image correspond to the pathological condition.

The set of one or more ultrasound image may be processed using the second neural network to generate the quantitative indication of the severity of the pathological condition and the second neural network may comprise: an object detection neural network configured to identify bounding boxes of continuous segments that correspond to the pathologic condition; and a model configured to generate a segmentation mask for each identified bounding box that indicates which pixels of that bounding box correspond to the pathological condition.

A pixel may correspond to the pathological condition if an intensity of the pixel is less than an intensity threshold.

Processing the set of one or more ultrasound image using the second neural network to generate the quantitative indication of a severity of the pathological condition may comprises: processing the set of one or more ultrasound image using the second neural network to generate one or more segmentation mask; and determining an area of the set of one or more ultrasound image corresponding to the pathological condition based on the one or more segmentation mask.

Processing the set of one or more ultrasound image using the second neural network to generate the quantitative indication of the severity of the pathological condition may further comprise, prior to determining an area of the set of one or more ultrasound image corresponding to the pathological condition based on the one or more segmentation mask, binarizing the segmentation mask by applying a threshold to each element of the segmentation mask.

Each segmentation mask may be confined to pixels of a corresponding ultrasound image corresponding to an ultrasound beam.

The set of one or more ultrasound image may be processed using the second neural network to generate the quantitative indication of the severity of the pathological condition and the second neural network may comprise a feature extractor neural network that is configured to receive one or more ultrasound images and output a value that represents a predicted severity of the pathological condition.

The method may further comprise comprising training the feature extractor neural network using a regression loss function.

The pathological condition may be free fluid in the torso.

The quantitative indication of the severity of the pathological condition may be an estimate of a quantity of the free fluid in the torso.

The method may further comprise repeating (a) to (c) for a second set of one or more ultrasound image of the torso, wherein the set of one or more ultrasound image presents a first view of the torso, and the second set of one or more ultrasound image presents a second, different, view of the torso.

Each of the first and second views may be one of a right upper quadrant view, left upper quadrant view, pelvic view and subxiphoid view.

The set of one or more ultrasound image may comprise at least one image from a B-mode ultrasound video.

The set of one or more ultrasound image may comprise at least one M-mode ultrasound image.

The method may further comprise obtaining the set of one or more ultrasound image using one or more ultrasound transducer.

The method may further comprise, prior to obtaining the set of one or more ultrasound image, obtaining one or more A-mode ultrasound image of the torso, and analyzing the one or more A-mode ultrasound image to determine whether the one or more ultrasound transducer is positioned over an obstructing structure.

The method may further comprise determining, from an output of a pressure sensor located proximal to the one or more transducer, whether a pressure applied to the torso exceeds a predetermined threshold during a duration of the obtaining of the set of one or more ultrasound image.

The method may further comprise determining, from an output of an accelerometer located proximal to the one or more transducer, whether an acceleration of the one or more transducer exceeds a predetermined threshold at any point during the obtaining of the set of one or more ultrasound image.

The method may further comprise determining, from an output of a gyroscope located proximal to the one or more transducer, whether a rotation of the one or more transducer exceeds a predetermined threshold at any point during the obtaining of the set of one or more ultrasound image.

A fourth aspect provides system for processing data to detect a pathological condition based on a set of one or more ultrasound image of a torso, the system comprising: a memory storing instructions; and at least one processor coupled to the memory, the at least one processor configured to execute the instructions to perform any of the methods described above.

According to some aspects, the present disclosure provides a nontransitory computer-readable medium storing computer-executable instructions. The computer-executable instructions, when executed, configure a processor to perform any of the computations and processes described herein.

## BRIEF DESCRIPTION OF THE DRAWINGS

The drawings included herewith are for illustrating various examples of articles, methods, and systems of the present specification and are not intended to limit the scope of what is taught in any way. In the drawings:

FIG. 1 is a block diagram of a first example computing system for automatically detecting a pathological condition in an anatomical region of interest from a set of one or more ultrasound image of the anatomical region of interest;

FIG. 2 is a block diagram of an example computer which may be used to implement all or a portion of the computing systems of FIGS. 1 and 6 or the methods of FIGS. 7-9;

FIG. 3A is a schematic diagram of an example amplitude-mode (A-mode) ultrasound signal;

FIG. 3B is a screenshot of an example brightness-mode (B-mode) ultrasound video;

FIG. 3C is a screenshot of an example motion-mode (M-mode) ultrasound image;

FIG. 4A is a schematic diagram illustrating an example beam shape for a linear ultrasound probe;

FIG. 4B is a schematic diagram illustrating an example beam shape for a curvilinear ultrasound probe;

FIG. 4C is a schematic diagram illustrating an example beam shape for a phased array ultrasound probe;

FIG. 5 is a schematic diagram illustrating an example method of generating an M-mode ultrasound image from a B-mode ultrasound video;

FIG. 6 is a block diagram of a second example computing system for automatically detecting a pathological condition in an anatomical region of interest from a set of one or more ultrasound image of the anatomical region of interest;

FIG. 7 is a flow diagram of an example method for automatically detecting a pathological condition in an anatomical region of interest from a set of one or more ultrasound image of the anatomical region of interest;

FIG. 8 is a flow diagram of a first example method of implementing the method of FIG. 7 using a first neural network to perform anatomical structure detection and second, different neural network to perform pathological condition detection; and

FIG. 9 is a flow diagram of a second example method of implementing the method of FIG. 7 using a single neural network to perform anatomical structure detection and pathological condition detection.

## DETAILED DESCRIPTION

While the FAST examination has proved invaluable in detecting intraperitoneal bleeding after abdominal trauma, it requires an expert to both (a) obtain ultrasound images corresponding to the four views, and (b) analyze the obtained ultrasound images to determine if the ultrasound images show intraperitoneal bleeding. It may be the same expert or different experts that perform (a) and (b). Requiring one or more experts to implement the FAST examination makes the FAST examination prone to time delays, if the required experts are not available, and/or human error. Accordingly, it would be desirable to be able to perform the FAST examination, and other similar ultrasound-based diagnosis methods, without such experts.

Accordingly, described herein are artificial intelligence (AI) - based computing systems and methods for detecting pathological conditions (e.g., free fluid) in an anatomical region of interest (e.g., a view of the torso) from a set of one or more ultrasound image of the anatomical region of interest. The described methods comprise processing a set of one or more ultrasound image using one or more neural networks to (1) determine whether a plurality of anatomical structures are present in the set of one or more ultrasound image; and (2) determine whether the pathologic condition is present. If it is determined, from the anatomical structure determination, that the one or more ultrasound image is suitable for detecting the pathological condition then the determination of whether the pathological condition is present may be output; otherwise, an indication that the set of one or more ultrasound image is not suitable for detecting the pathological condition may be output.

In some cases, a set of one or more ultrasound image may be suitable for detecting a pathological condition in an anatomical region of interest if the set of one or more ultrasound image adequately show the anatomical region of interest (indicating that the ultrasound probe was placed in the correct position when the set of one or more ultrasound image was captured). A set of one or more ultrasound image may be deemed to adequately show the anatomical region of interest if the set of one or more ultrasound image show a certain combination of anatomical features. For example, as described in more detail below, a set of one or more ultrasound image of the RUQ may be deemed suitable for detecting free fluid if the set of one or more ultrasound image display the liver and right kidney.

Automatically assessing the suitability of ultrasound images for detecting a pathological condition and detecting the pathological condition from suitable ultrasound images may mean that detection of a pathological condition from ultrasound images can be performed by an individual with less training or understanding of gathering and/or interpreting ultrasound images. This may allow such pathological condition detection to be performed by more individuals, which may allow the pathological condition detection to be performed more quickly since the detection does not have to wait for an expert in ultrasound image gathering and/or interpretation.

Automatically assessing the suitability of ultrasound images and detecting the pathological condition from suitable ultrasound images may also increase the accuracy of the detection (e.g., diagnosis), which may increase the safety of the detection. Specifically, not only does this reduce human error associated with obtaining and analyzing ultrasound images, but it ensures that the pathological condition detection is only provided to the user if the set of ultrasound images is of sufficient quality to make an accurate detection. Specifically, ultrasound images are preferably acquired in a correct manner and feature anatomical structures the pathological condition detection neural network has been trained on. The absence of, or the positions of, anatomical structures of interest over the course of a set of images are valuable in determining if (1) the ultrasound probe is positioned correctly for detection of the pathological condition, and (2) if the ultrasound operator was moving the probe too quickly or erratically to collect quality ultrasound images suitable for interpretation.

In some cases, there may be a first neural network that is configured and trained to perform the anatomy detection, and a second, different, neural network that is configured and trained to perform the pathological condition detection. In these cases, the set of one or more ultrasound image may be first processed by the first neural network to predict the presence and/or position of each of a plurality of anatomical structures in the set of one or more ultrasound image. It may then be determined, from the predicted presence and/or position of the plurality of anatomical structures, whether the set of one or more ultrasound image is suitable for detecting the pathological condition. If it is determined that the set of one or more ultrasound image is suitable for detecting the pathological condition, then the set of one or more ultrasound image may be processed by the second neural network to generate a prediction of a presence of the pathological condition and/or a quantitative indication of a severity of the pathological condition. The prediction of the presence of the pathological condition and/or the quantitative indication of the severity of the pathological condition may then be output (e.g., to a display). Accordingly, the prediction of the presence of the pathological condition and/or the quantitative indication of the severity of the pathological condition is only output if it is determined that the set of one or more ultrasound image is suitable for detecting the pathological condition.

In some cases, where the one or more neural networks comprise a first neural network that is configured and trained to perform the anatomy detection, and a second, different neural network that is configured and trained to perform the pathological condition detection, the second neural network may be configured to only receive and process the set of one or more ultrasound image of the anatomical region of interest (e.g., torso); yet in other cases, the second neural network may be configured to receive and process the set of one or more ultrasound image of the toros and anatomical data based on the output of the first neural network (e.g., the predicted presence and/or location of each of the plurality of anatomical structures in the set of one or more ultrasound image of the anatomical region of interest (e.g., torso)). As described in more detail below, by providing the second neural network with information indicating the location or position of anatomical structures surrounding the areas where the pathological condition (e.g. free fluid) may exist, the second neural network may be able to localize the areas where the pathological condition (e.g., free fluid) may exist and provide more correct boundaries therefor.

In other cases, there may be a single neural network that is configured and trained to perform both the anatomy detection and the pathological condition detection, the single neural. Specifically, a single neural network may be configured and trained to receive a set of one or more ultrasound image of the anatomical region of interest (e.g., torso) and generate a prediction of the presence and/or position of each of a plurality of anatomical structures in the set of one or more ultrasound image; and generate a prediction of the presence of the pathological condition and/or a quantitative indication of a severity of the pathological condition. In these cases, the anatomy detection and pathological condition detection are performed at the same time or concurrently, and the pathological condition detection (e.g., the prediction of the presence of the pathological condition and/or quantitative indication of the severity of the pathological condition) is only output if it is determined from the anatomy detection (e.g., the prediction of the presence and/or position of each of the plurality of anatomical structures) that the set of one or more ultrasound image is suitable for detection of the pathological condition. As will be described in more detail below, testing has shown that a single neural network configured to perform both anatomy detection and pathological condition detection may reduce the total inference time and improve the ability of the neural network to accurately detect the pathological condition (e.g. free fluid).

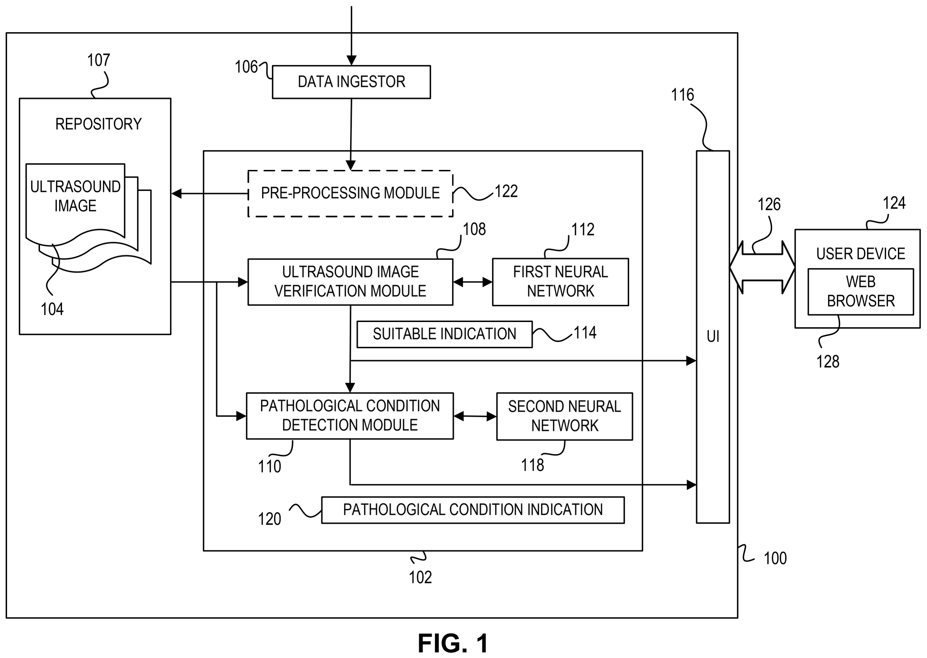

Reference is first made to FIG. 1 which illustrates a first example computing system 100 for detecting a pathological condition (e.g., free fluid) in an anatomical region of interest (e.g., a region of the torso) from a set of one or more ultrasound image of the anatomical region of interest. In this example, there is a first neural network configured and trained to perform anatomy detection and a second, different, neural network configured and trained to perform pathological condition detection.

The example computing system 100 comprises an assessment module 102 that is configured to receive a set of one or more ultrasound image 104 of an anatomical region of interest (e.g., a region of the torso) and (1) automatically determine if the set of one or more ultrasound image 104 is suitable for detecting the pathological condition; and (2) if the set of one or more ultrasound image 104 is deemed suitable for detecting the pathological condition, automatically determine from the set of one or more ultrasound image 104 if the pathological condition is present.

In some cases, one or more components of the computing system 100 may be implemented by one or more computers within the computing system 100, such as, but not limited to, computer 200 described below with respect to FIG.

2.

The set of one or more ultrasound image 104 may comprise a single ultrasound image or a plurality of ultrasound images. An ultrasound image, which may as be referred to as a sonography, is a picture of the inside of a patient's body that is created using high-frequency sound waves. Specifically, a sound wave probe sends high-energy sound waves into the body. The sound waves bound off tissues, organs etc., creating echoes. The probe captures the echoes and converts them into electrical energy which a processor turns into an image. The term ultrasound image includes but is not limited to, a motion mode (M-mode) image and an image (e.g., frame) extracted from a brightness mode (B-mode) video. Each of these types of ultrasound images are described in more detail below. In some cases, where the set of one or more ultrasound image comprises a plurality of ultrasound images the plurality of ultrasound images may form a video, such as, but not limited to, a B-mode video. In such cases, the set of one or more ultrasound images may be transmitted and/or stored in a single file. In other cases, where the set of one or more ultrasound image comprises a plurality of ultrasound images the plurality of ultrasound images may be transmitted or stored in a plurality files (e.g., there may be a separate file for each ultrasound image).

In some cases, the set of one or more ultrasound image 104 may be received, e.g., from an ultrasound machine, or a set of one or more ultrasound transducer, via a data ingestor 106. The data ingestor 106 may be configured to actively retrieve the set of one or more ultrasound image 104 or the data ingestor 106 may be configured to passively receive the set of one or more ultrasound image 104. In some cases, the set of one or more ultrasound image 104 received by the data ingestor 106 may be processed directly by the assessment module 102. In other cases, the data ingestor 106 may be configured to store the received set of one or more ultrasound image 104 in a repository 107 and the assessment module 102 may be configured to retrieve the set of one or more ultrasound image 104 from the repository 107. The repository 107 may be any mechanism or device, such as, but not limited to, memory, that can be used to store digital information.

As described above, the assessment module 102 is configured to receive a set of one or more ultrasound image 104 of an anatomical region of interest (e.g., a region of the torso) and (1) automatically determine if the set of one or more ultrasound image 104 is suitable for detecting the pathological condition; and (2) automatically determine from the set of one or more ultrasound image 104 if the pathological condition is present. In the example shown in FIG. 1, the assessment module 102 comprises an ultrasound image verification module 108 that is configured to automatically determine whether the set of one or more ultrasound image 104 is suitable for detecting the pathological condition, and a pathological condition detection module 110 that is configured to, if it determined by the ultrasound image verification module 108 that the set of one or more ultrasound image 104 is suitable for detecting the pathological condition, determine, from the set of one or more ultrasound image 104, whether the pathological condition is present.

In the examples described herein the ultrasound image verification module 108 is configured to determine whether the set of one or more ultrasound image 104 is suitable for detecting the pathological condition by determining whether the set of one or more ultrasound image 104 adequately shows the anatomical region of interest. A set of one or more ultrasound image 104 may be deemed to adequately show the anatomical region of interest if the set of one or more ultrasound image 104 shows a combination of anatomical features associated with the anatomical region of interest. In some cases, the ultrasound image verification module 108 may determine whether the set of one or more ultrasound image 104 shows a desired combination of features by (i) processing the set of one or more ultrasound image 104 using a first neural network 112 to generate a prediction of the presence and/or location of each of a plurality of anatomical structures in the set of one or more ultrasound image 104, (ii) determining whether the set of one or more ultrasound image 104 is suitable for detecting the pathological condition based on the predicted presence and/or location of the plurality of anatomical structures.

For example, if the anatomical region of interest is the RUQ view used in the FAST examination, then it may be determined that the set of one or more ultrasound image 104 is suitable for detecting a pathological condition if the liver and right kidney are present. Different anatomical regions of interest may be associated with different combinations of anatomical structures. For example, each of the four different views of the torso used in the FAST examination may be associated with a different combination of anatomical structures.

In some cases, the ultrasound image verification module 108 may be configured to use the first neural network 112 to generate a prediction of the presence of each of the plurality of anatomical structures in the set of one or more ultrasound image 104 (e.g., a prediction of whether each of the plurality of anatomical structures is in the set of one or more ultrasound image 104). In such cases, the first neural network 112 may comprise a multi-label image classifier neural network, that is configured to receive one or more ultrasound images and generate a multi-element vector in which each element of the multi-element vector comprises the prediction of the presence of one of the plurality of anatomical structures in the received one or more ultrasound image. The multi-label classifier neural network may, for example, be a convolutional neural network or a vision transformer neural network.

In other cases, the ultrasound image verification module 108 may be configured to use the first neural network 112 to generate a prediction of the location of each of a plurality of anatomical structures in the set of one or more ultrasound image 104. In such cases, the first neural network 112 may comprise an object detection neural network.

In some cases, the first neural network 112 may be configured to process one ultrasound image at a time - i.e., the first neural network 112 may be configured to receive a single ultrasound image and generate a prediction as to the presence and/or location of the plurality of anatomical structures in that ultrasound image. In such cases, where the set of one or more ultrasound image comprises a plurality of ultrasound images the ultrasound image verification module 108 may be configure to process each ultrasound image of the plurality of ultrasound images using the first neural network 112 to generate a prediction of the presence and/or position of each of the plurality of anatomical structures in that ultrasound image, and generate a prediction of the presence and/or position of each of the plurality of anatomical structures for the set of one or more ultrasound image 104 based on the per image predictions (e.g. by combining the per image predictions). In other cases, the first neural network 112 may be configured to process a plurality of ultrasound images as whole (e.g., as a video input) such that the first neural network 112 can be used to generate a prediction for the set of one or more ultrasound image in one forward pass of the first neural network 112.

Example implementations of the ultrasound image verification module 108 and the first neural network 112 are described in detail below.

In some cases, as shown in FIG. 1, the ultrasound image verification module 108 may be configured to output an indication (which may be referred to as the suitable indication 114) of whether the set of one more ultrasound image 104 is suitable for detecting the pathological condition. In some cases, the suitable indication 114 may be output to a user, e.g., via a user interface 116 of the computing system 100.

The pathological condition detection module 110 is configured to, in response to the ultrasound image verification module 108 determining that the set of one or more ultrasound image 104 is suitable for detecting the pathological condition, automatically determine from the set of one or more ultrasound image, whether the pathological condition is present (the determination may be referred to as the pathological condition indication 120). This may be implemented by the pathological condition detection module 110 by processing the set of one or more ultrasound image 104 using a second neural network 118 to generate a prediction of the presence of the pathological condition and/or a quantitative indication of the severity of the pathological condition.

In some cases, the set of one or more ultrasound image 104 is processed using the second neural network 118 to generate a prediction of the presence of the pathological condition. In such cases, the second neural network may be a classifier neural network. In some cases, the classifier neural network may be a single class classifier neural network that is configured to receive one or more ultrasound images and generate a single value that indicates whether the pathological condition is predicted to be present based on the one or more ultrasound images. In other cases, the classifier neural network may be a multi-class classifier neural network that is configured to receive one or more ultrasound images and generate a plurality of values that indicate varying degrees of detection of the pathological condition For example, the multi-class classifier network may be configured to classify the set of one or more ultrasound image as (1) the pathological condition is present, (2) the pathological condition is possibly present, or (3) the pathological condition is not present.

In some cases, the set of one or more ultrasound image 104 is processed using the second neural network 118 to generate quantitative indication of the severity of the pathological condition. Where the pathological condition is free fluid then the quantitative indication of the severity of the pathological condition may be an indication of the amount of free fluid detected. In some cases, the second neural network may comprise a semantic segmentation neural network that is configured to receive an ultrasound image and generate a segmentation mask that indicates which pixels of the ultrasound image correspond to the pathological condition. In other cases, the second neural network may comprise an object detection neural network that is configured to receive an ultrasound image and identify bounding boxes of continuous segments that correspond to the pathological condition (e.g., contain free fluid) and a model configured to generate a segmentation mask for each identified bound box that indicates which pixels of that bounding box correspond to the pathological condition. In either case, the pathological condition detection module 110 may be configured to determine an area of the set of one or more ultrasound image corresponding to the pathological condition based on the one or more segmentation mask generated by the second neural network 118.

In some cases, instead of using the second neural network 118 to generate one or more segmentation masks and then generating a quantitative indication (e.g., area) from the one or more segmentation masks, the second neural network may comprise a feature extractor neural network that is configured to receive one or more ultrasound images and output a value that represents the predicted severity of the pathological condition.

Example implementations of the pathological condition detection module 110 and the second neural network 118 are described in detail below.

In some cases, as shown in FIG. 1, the pathological condition indication 120 may be provided to a user via a user interface 116 of the computing system 100. Specifically, the pathological condition indication 120 may be provided to a user device 124 that is connected over a data communication link 126 to the user interface 116. For example, the user may receive the pathological condition indication 120 via a web browser 128 or some other application that operates on the user device 124. In some cases, the ultrasound probe, the user device and/or the computing system 100 may be integrated into a single device.

**Example Computer**

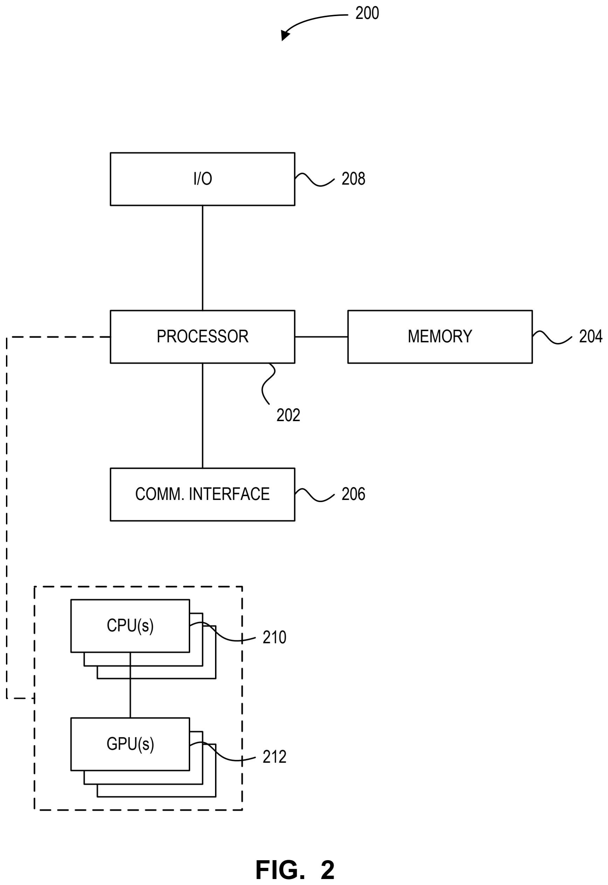

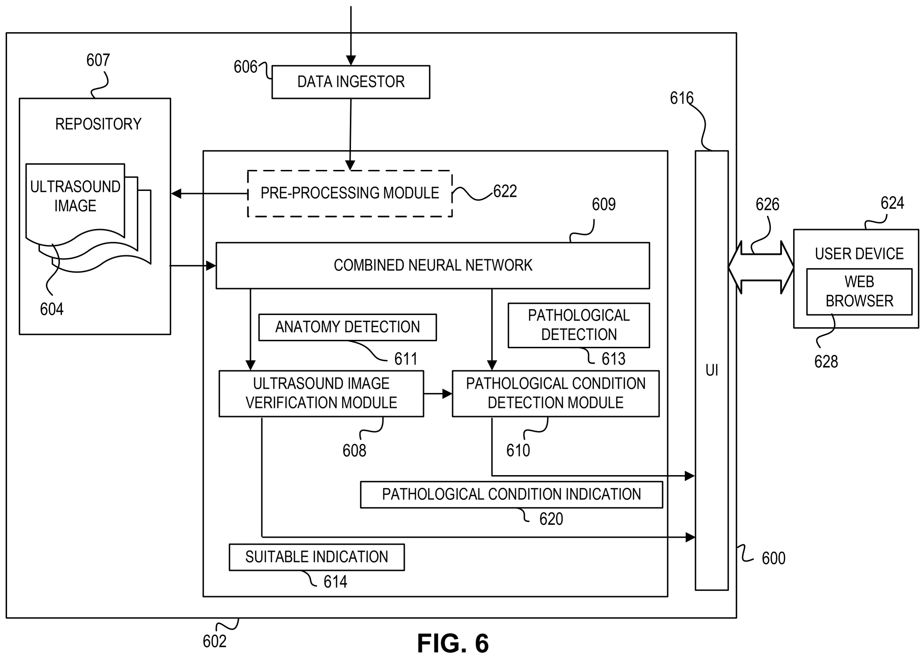

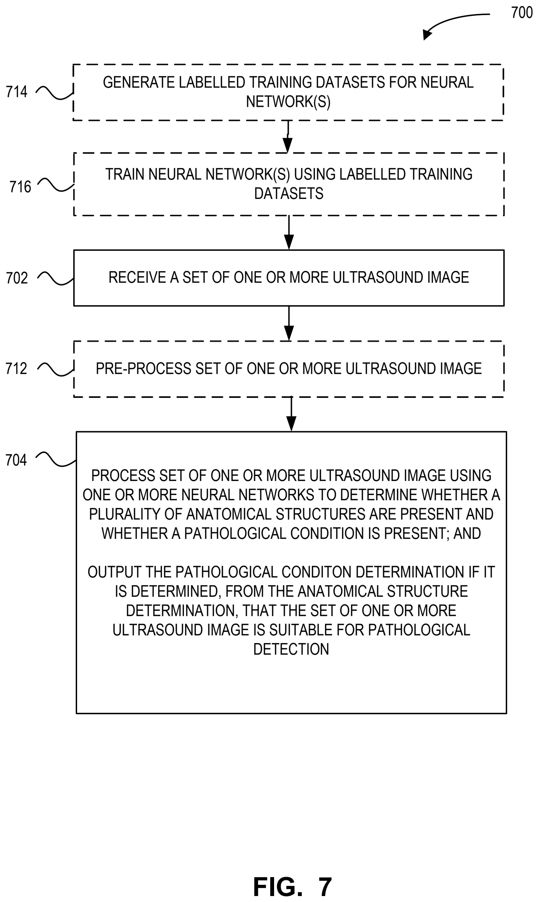

Reference is now made to FIG. 2, which illustrates a simplified block diagram of an example computer 200. The computer 200 of FIG. 2 may be used to implement all, or a part of, the computing system 100 of FIG. 1, the computing system 600 of FIG. 6, user device 124 of FIG. 1 and/or any of methods 700, 800, 900 of FIG. 7, 8 and 9 respectively. Computer 200 has at least one processor 202 operatively coupled to at least one memory 204, at least one communications interface 206 (also herein called a network interface), and at least one input/output device 208.

The at least one memory 204 includes a volatile memory that stores instructions executed or executable by processor 202, and input and output data used or generated during execution of the instructions. Memory 204 may also include non-volatile memory used to store input and/or output data - e.g., within a database - along with program code containing executable instructions.

Processor 202 may transmit or receive data via communications interface 206, and may also transmit or receive data via any additional input/output device 208 as appropriate.

In some cases, the processor 202 includes a system of central processing units (CPUs) 210. In some other cases, the processor includes a system of one or more CPUs and one or more Graphical Processing Units (GPUs) 212 that are coupled together. For example, the first and/or second neural network 112, 118 may be implemented on CPU and/or GPU hardware, such as the system of CPUs 210 and GPUs 212.

**Types of Ultrasound Information**

As described above, ultrasound information, which may also be referred to as sonography information, is information about the inside of a patient's body that is obtained using high-frequency sound waves. Specifically, a sound wave probe sends high-energy sound waves into the body. The sound waves bound off tissues, organs etc., creating echoes. The probe captures the echoes and converts them into electrical energy which a processor turns into a signal, a video, or an image.

There are several standard formats for ultrasound information or ultrasound data streams, which include, but are not limited to, amplitude mode (A-mode) signals, brightness mode (B-mode) videos and motion mode (M-mode) images.

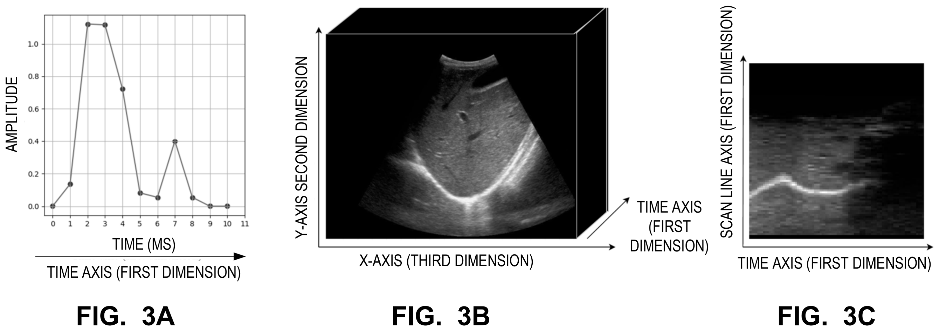

A-mode signals are the simplest type of ultrasound information. An A-mode signal is one-dimensional (1D) and comprises a series of values, that may be represented as a vector $a \in R_+^D$, where $a[t]$ is the amplitude of the voltage recorded by an ultrasound transducer received at time $t$, where $D$ is the total number of time steps at which the amplitude is sampled along the scan line. Each value in the vector is representative of the strength of the echo that arrives at time step $t$. FIG. 3A shows an example A-mode image.

Traditional B-mode videos are a series of two-dimensional (2D) greyscale images where the brightness of each pixel value indicates the amplitude of the reflected waves received by the transducer. To be used as an input to a neural network a traditional B-mode video can be represented as a 3D tensor $\boldsymbol{B} \in R^{N \times H \times W}$, where $N$ is the number of frames in the video, $H$ is the height of the images, and $W$ is the width of the images. In some cases, the pixel values may be in the range [0,255]. In other cases, the pixel values may be in a different range. FIG. 3B shows an example B-mode video.

B-mode colour videos are a variation of traditional B-mode videos that use colour to enhance the video. For example, instead of comprising a sequence of greyscale images, a B-mode colour video may comprise a sequence of RGB images. To be used as an input to a neural network a B-mode colour video can be represented as a 4D tensor $B \in R^{N \times H \times W \times 3}$. The term "B-mode video" is used herein to include both traditional B-mode videos and B-mode colour videos.

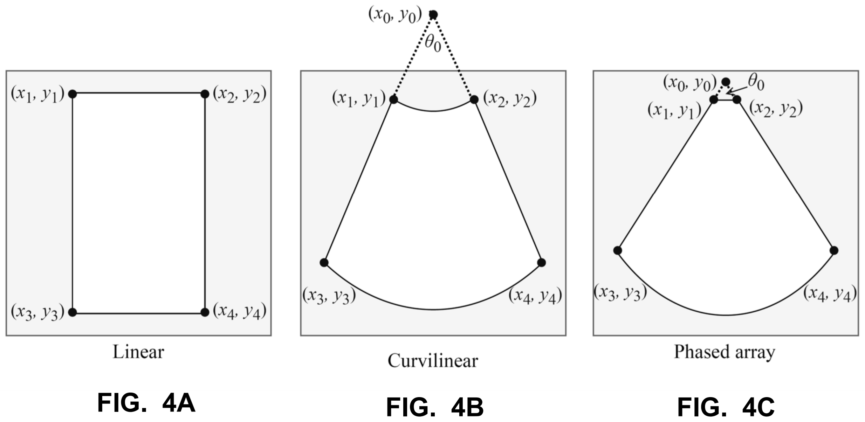

The ultrasound beams generally manifest in a B-mode video in a shape that corresponds to the type of probe used to acquire the B-mode video. For example, a phased array probe generally induces the beam shape shown in FIG. 4C, a linear probe generally induces the beam shape shown in FIG. 4A, and a curvilinear probe generally induces the beam shape shown in FIG. 4B. These are example probe types and corresponding beam shapes and other probes may have other configurations which may result in other beam shapes.

The vertices of the beam shapes are identified by four co-ordinates: $p_1 = (x_1, y_1)$, $p_2 = (x_2, y_2)$, $p_3 = (x_3, y_3)$, and $p_4 = (x_4, y_4)$. FIGS. 4A, 4B and 4C show how the four co-ordinates map to the beam shapes shown therein. As can be seen in FIGS. 4B and 4C, additional features are used to describe the beam shapes for curvilinear and phased array probes. Specifically, the radius and sector angle (i.e., field of view) of the beam are identified by $r$ and $\theta_0$ respectively; and an additional co-ordinate $p_0 = (x_0, y_0)$ is defined as the point of intersection of the left and right linear bound of the beam. These lines meet at the sector angle $\theta_0$.

A traditional M-mode image is an image that depicts the evolution over time of the amplitudes of echoes received by the ultrasound probe along a single scan line at various depths. To be used as an input to a neural network, an M-mode image can be represented as a 2D tensor $M \in R^{H \times N}$. An example M-mode image is shown in FIG. 3C.

A-mode colour images are a variation of traditional M-mode images that use colour to enhance the image. For example, instead of the image being in greyscale images, an M-mode colour image may be an RGB image. To be used as an input to a neural network an M-mode colour image can be represented as a 3D tensor $M \in R^{H \times N \times 3}$, The term "M-mode image" is used herein to include both traditional M-mode images and M-mode colour images.

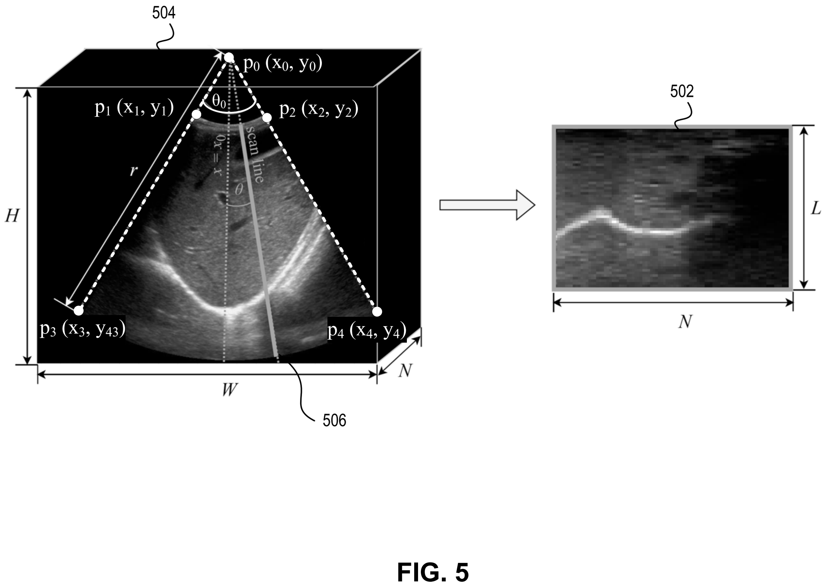

In some cases, as shown in FIG. 5, an M-mode image 502 may be generated from a previously obtained B-mode video $B$ 504 by specifying a scan line 506 in the image plane and taking all pixel values at $B[i,j, k]$ for all $i \in [0, N - 1]$ and all $(j, k)$ that lie on the scan line 506 and that are contained within the ultrasound beam. An M-mode scan line 506 is any line segment that is colinear with a line intersecting a transducer element.

In some cases, if a neural network (e.g., the first neural network, second neural network or combined neural network) is configured to process M-mode images, the M-mode images used to train the neural network may be M-mode images obtained from a previously acquired B-mode video. In some cases, the scan line used to obtain such M-images may have endpoints located at the top and bottom of the ultrasound beam and satisfies the following: (1) If the probe type is linear, the scan line is vertical. If the probe type is curvilinear or phase array, the scan line is colinear with a line that originates from the point of intersection of the left and right bounds of the beam (i.e., $p_0$). That is, for point $p_i$ that lies on the circular top bound of the beam, the scan line consists of all points contained within the beam that lie on the line that passes through $p_0$ and $p_i$. (2) The scan line is within the horizontal lines of the beam. These constraints are intended to mimic the path of a single ultrasound scan line, such as scan line 506 shown in FIG. 5. Specifically, the scan line starts from the transducer and travels outward radially (for phased area or curvilinear ultrasound probes) or vertically (for a linear ultrasound probe).

**Pre-Processing of Images**

In some cases, the assessment module 102 may comprise a pre-processing module 122 that is configured to pre-process a set of one or more ultrasound image before it is processed by the ultrasound image verification module 108 and, optionally, the pathological condition detection module 110.

In some cases, where the received set of one or more ultrasound image is in colour (e.g., the set of one or more ultrasound image comprises one or more images or frames of an RGB B-mode video or one or more RGB M-mode images) the pre-processing module 122 may be configured to convert the received set of one or more ultrasound image into greyscale. Whether or not a received set of one or more colour ultrasound images is converted to greyscale may depend on the configuration of the first and second neural networks 112, 118. For example, if the first and second neural networks are configured to process greyscale images then a set of one or more colour ultrasound images may be converted to greyscale. If, however, the first and second neural networks are configured to process colour images, then a set of one or more more colour ultrasound images may not be converted to greyscale. Since a tensor representing a greyscale ultrasound image or a set of greyscale ultrasound images will be smaller than a tensor representing a colour ultrasound image or a set of colour ultrasound images, training a neural network, such as the first and second neural networks 112, 118 of FIG. 1, to process greyscale ultrasound image(s), may allow the neural networks to be smaller and thus require less memory to store and less processing power and resources to implement. It may also, or alternatively, allow the neural networks to be more generalizable as they would not be at risk of suffering from distribution shift if a new chroma profile is encountered at test time.

In some cases, the pre-processing module 122 may also, or alternatively, be configured to remove extraneous information from the received set of one or more ultrasound image. Specifically, the pixels of an ultrasound image conveying information in the ultrasound beam may be surrounded by a, generally, black margin of varying size that contains graphical entities that communicate information, such as, but not limited to, manufacturer logos/content, patient information, and/or clinical notes. In some cases, the pre-processing module 122 may be configured to remove such extraneous information from an ultrasound image by generating a mask that represents the pixels of the ultrasound image that fall in the ultrasound beam. Then a uniform colour can be applied to all pixels that fall outside the mask to remove the extraneous information. The ultrasound images may then be cropped to focus on the region of interest. For example, the ultrasound images may be cropped using the smallest rectangle that completely encloses the ultrasound beam.

The masking approach may be generalizable to ultrasound beams of varying widths and positions, as different machines have different interfaces. To facilitate finding a suitable mask, the largest continuous contour may be detected using computer vision techniques for a plurality of frames in a video, and the frame with the largest contour may be used for calculations of the ultrasound beam. The two linear edges may be derived by finding every point on the contour that is both the top-most and left- or rightmost in every column and row, and finding two lines of best fit for both sets of points. The bottom circular edge may then be calculated using the intersection of these two lines as the vertex, and fitted to all the bottom-most points on the contour. A mask may be generated using these edges and subsequently applied to every ultrasound image in the set of one or more ultrasound images. If a matching ultrasound beam is not found within empirically determined limits due to poor-quality beams, then the process may revert to using the contour as the mask. This approach may facilitate removing information outside of the ultrasound beam and preserving information that may have been left out by the contour. In some cases, however, text or interface artifacts contained within the beam portion of the image may be retained.

An example method for removing extraneous information from an ultrasound image via a mask, which may be implemented by the pre-processing module 122, is described in the Applicant's US Patent Application No. 18/935,011, which is herein incorporated by reference in its entirety.

**Ultrasound Image Verification Module and First Neural Network**

As described above, the ultrasound image verification module 108 is configured to determine whether the set of one or more ultrasound image 104 is suitable for detecting the pathological condition by determining whether the set of one or more ultrasound image 104 adequately shows the anatomical region of interest. The ultrasound image verification module 108 is configured to determine that a set of one or more ultrasound image adequately shows the anatomical region of interests by processing the set of one or more ultrasound image 104 using the first neural network 112 to generate a prediction of the presence and/or location of each of a plurality of anatomical structures in the set of one or more ultrasound image 104, then determining whether the set of one or more ultrasound image 104 is suitable for detecting the pathological condition based on the predicted presence and/or location of the plurality of anatomical structures.

For example, an anatomical region of interest may be associated with a set of orienting anatomical structures $S = s_0, s_1, \ldots, s_K$. A Boolean vector $s \in \{0,1\}^K$ may be used to represent the presence of each of the orienting anatomical structures in a set of one or more ultrasound image. Specifically, $s[i] = 1$ indicates the presence of the $i^{th}$ orienting structure and $s[i] = 0$ indicates its absence. The anatomical region of interest may also be associated with a clinically based function $\beta : \{0,1\}^K \to X$ that maps the presence of orienting structures (i.e., $s$) to a decision $X$ indicating the suitability of the set of one or more ultrasound image for detecting the pathological condition. As described in more detail below, the decision $X$ may be a single value that indicates whether the set of one or more ultrasound image is suitable for detecting the pathological condition, or the decision $X$ may be multiple values that indicate the degree of certainty regarding the suitability of the set of one or more ultrasound image for detecting the pathological condition. For example, the decision $X$ may be a ternary decision $\{0,1,2\}$ that indicates whether the set of one or more ultrasound image is (0) suitable for detecting the pathological condition, (1) is not suitable for detecting the pathological condition, or (2) contains an anatomically impossible combination of orienting anatomical structures.

For example, if the anatomical region of interest is the RUQ view of the torso used in the FAST examination, then the ultrasound image verification module 108 may be configured to predict the presence of the liver and right kidney (and in some cases the hepatorenal recess) and determine that the set of one or more ultrasound image 104 is suitable for detecting a pathological condition if it is predicted that the liver and right kidney (and in some cases the hepatorenal recess) are present. Different anatomical regions of interest may be associated with different orienting anatomical structures and/or different clinically based functions $\beta$. For example, each of the four different views of the torso used in the FAST examination may be associated with different orienting anatomical structures and/or different clinically based functions $\beta$. For example, the orienting anatomical structures for the RUQ view may be the spleen, right kidney and optionally the hepatorenal recess; the orienting anatomical structures for the LUQ view may be the left kidney, spleen and diaphragm, and optionally the splenorenal recess; the orienting anatomical structures for the pelvic view may be the bladder and rectum for males, and the bladder and uterus for females; and the orienting anatomical structures for the subxiphoid view may be the heart, pericardium, and optionally the liver.

In some cases, the ultrasound image verification module 108 may be configured to use the first neural network 112 to generate a prediction for s for the set of one or more ultrasound image 104 and then determine, from the prediction for s, whether the set of one or more ultrasound images is suitable for detecting the pathological condition. In other words, in some cases, the ultrasound image verification module 108 may be configured to use the first neural network 112 to generate a multi-element vector in which each element of the multi-element vector comprises a prediction of the presence of one of the plurality of orienting anatomical structures and then determine, from that multi-element vector, whether the set of one or more ultrasound image is suitable for detecting the pathological condition.

In these cases, the first neural network 112 may be configured to receive one or more ultrasound images and generate a prediction for s for the received one or more ultrasound images. In such cases, the first neural network 112 may be a multi-label or multi-class image classifier neural network. For example, the first neural network 112 may be a K-class image classifier neural network, where K is the number of orienting anatomical structures. The multi-label classifier neural network may, for example, be a convolutional neural network, a vision transformer neural network, or a novel neural network that comprises a combination of operations such as, but not limited to, pooling, nonlinear activation functions, batch normalization, sample normalization, attention, residual connections, and/or linear projections. The multi-label classifier neural network may comprise a submodule adopted from pre-existing architectures such as, but not limited to, VGG-16, MobileNet, Efficient-Net or ViT.

In some cases, the output of the neural network may comprise a prediction for s (a K-dimensional vector, where K is the number of orienting anatomical structures) wherein each element of the s is in the range [0,1]. In some cases, the final layer of the first neural network 112 is a fully connected layer with K-nodes that may or may not be followed by an activation function, that is configured to generate the prediction for s. The fully connected layer, by connecting every neuron in the input to every neuron in the output, performs high-level reasoning and decision making. In some cases, sigmoid activation (i.e., $\sigma(x) = 1 / (1 + e^{-kx})$, $k \in R$) may be applied to each node of the fully connected layer. A sigmoid activation squashes the output to a probability value between 0 and 1 which can be interpreted as the probability of the input belonging to a particular class.

In some cases, all or a portion of the clinically based function $\beta$ may be integrated into the first neural network. For example, constraints of the clinically based function $\beta$ may embedded within the first neural network via a series of gating mechanisms applied to the output of, for example, the fully connected layer described above (i.e., the final fully connected layer of the first neural network). Specifically, instead of each element of the final vector s being computed solely from its corresponding output from the fully connected layer (pre-activation), in some cases, the different elements of the final vector s may be computed using the values of other elements of the output of the fully connected layer. For example, if the first and second orienting structures can never be present in the same image, then if $\hat{y}$ is the pre-activation output of the fully connected layer, then confidences or predictions for orienting anatomical structures 1 and 2 could be computed as $\hat{s}_1 = \sigma(\hat{y}_1)$ and $\hat{s}_2 = \sigma(\hat{y}_2)(1 - \hat{s}_1)$.

In some cases, the first neural network 112 may be configured to receive and process a plurality of ultrasound images as a whole (e.g., as one input tensor) - e.g., a plurality of images or frames of a B-mode video, or a plurality of related M-mode images - and generate a prediction for s for the plurality of ultrasound images. A plurality of related M-mode images may, for example, comprise M-mode images acquired synchronously in parallel from distinct transducers in an ultrasound probe or M-mode images indexed from the same B-mode video. In these cases, where the set of one or more ultrasound images comprises a plurality of ultrasound images, a prediction for s for the plurality of ultrasound images can be generated by the ultrasound image verification module 108 via one forward pass of the first neural network 112.

In other cases, the first neural network 112 may be configured to receive and process a single ultrasound image at a time and generate a prediction for s for the received ultrasound image. In these cases, where the set of one or more ultrasound image comprises a plurality of ultrasound images, the ultrasound image verification module 108 may be configured to generate a prediction for s for the plurality of ultrasound images as whole by processing each ultrasound image of the plurality of ultrasound images using the first neural network 112 to generate a prediction for s for the received ultrasound image and then generating a prediction for s for the plurality of ultrasound images as whole from the per image predictions for **s**.

The prediction of **s** for each ultrasound image comprises a prediction $\hat{s}_i$ for each orienting anatomical structure $i$. In some cases, the prediction for s for the plurality of ultrasound images as a whole may be generated by the ultrasound image verification module 108 by computing, for each orienting anatomical structure $i$, the mean across all individual image predictions for $\hat{s}_i$, then applying a classification threshold $c_i$. In other cases, the prediction for s for the plurality of ultrasound images as a whole may be generated by the ultrasound image verification module 108 by applying a moving average (with window size $w_i \in N$) to each $\hat{s}_i$ across the plurality of ultrasound images. If there exists at least $\tau_i \in N$ ultrasound images from which $\hat{s}_i \geq c_i$, the orienting anatomical structure may be predicted to be present in the plurality of ultrasound images; otherwise, it is predicted to be absent. Values for each $c_i$, $w_i$ and/or $\tau_i$ may be determined by engaging in a grid search using a validation or calibration dataset. In some cases, there may be a different $c_i$, $w_i$ and/or $\tau_i$ for each orienting anatomical structure.

The first neural network 112 may be configured to receive and process one or more B-mode images or one or more M-mode images.

Where the first neural network 112 is configured to receive one or more ultrasound images of a particular mode and generate a prediction for s for the received ultrasound image, the first neural network 112 may be trained on ultrasound images of that particular mode with annotations for the presence of each of the plurality of anatomical structures. For example, if the first neural network 112 is configured to receive and process one B-mode image at a time, then the first neural network may be trained on B-mode images with annotations for the presence of each of the plurality of annotating structures. Similarly, if the first neural network 112 is configured to receive and process a plurality of M-mode images at a time then the first neural network may be trained on sets of M-mode images with annotations for the presence of each of the plurality of annotating structures. Such first neural networks 112 may be trained using appropriate loss functions, such as, but not limited to, binary cross-entropy or focal loss. Custom regularizers may also be developed and integrated in the loss function that consist of Lagrangian multipliers that constrain solutions to plausible values of s.

In some cases, instead of the ultrasound image verification module 108 using the first neural network 112 to generate a prediction of each of a plurality of anatomical structures in the set of one or more ultrasound image, the ultrasound image verification module 108 may be configured to use the first neural network 112 to generate a prediction of the position or location of each of the plurality of anatomical structures in the set of one or more ultrasound image 104 and determine whether the set of one or more ultrasound image 104 is suitable based on the detected locations. In such cases, the first neural network 112 may comprise an object detection neural network that is configured to receive one or more ultrasound images and output bounding boxes of objects identified therein and a classification of each identified object.

The object detection neural network may be configured to implement any suitable bounding box detection method, such as, but not limited to YOLO (You Only Look Once) and SSD (single shot detector). Such neural networks may be configured to process one or more B-mode images or one or more M-mode images.

Where the first neural network is configured to perform object detection, the first neural network 112 may be configured to process one ultrasound image at a time or the first neural network 112 may be configured to process multiple ultrasound images at a time (e.g., as a whole). If the first neural network 112 is configured to receive and process one ultrasound image at a time and the set of one or more ultrasound image comprises a plurality of ultrasound images, then the ultrasound image verification module 108 may be configured to process each ultrasound image using the first neural network 112 to generate location information for each image and determine whether the set of one or more image is suitable for detecting the pathological condition from the location information for each of the images. In some cases, bounding box tracking methods may be applied to bounding box predictions to produce confident predictions for orienting anatomical structures for a plurality of ultrasound images. In these case, the first neural network 112 may be used to generate a list of bounding boxes that that correspond to predicted objects. After passing this output to a bounding box tracking module, each box may be assigned an identifier that estimates which object it belongs to. For example, if there are two orienting anatomical structures then there may be two bounding box predictions per ultrasound image, where each of those bounding boxes would be given an identifier. Boxes that have the same identifier in different frames are meant to refer to the same anatomical structure.

Where the first neural network 112 is configured to perform object detection, the first neural network may be trained on ultrasound images of a particular type that have bounding box annotations for each orienting anatomical structure.

In other cases, instead of the first neural network 112 being an objection detection model that predicts the position or location of each of the plurality of anatomical structures in the set of one or more ultrasound image by identifying bounding boxes of objects identified therein and classifying each identified object; the first neural network may be a semantic neural network that predicts the position or location of each of the plurality of anatomical structures by assigning class labels to individual pixels. The output of a segmentation model is one or more pixel-level maps that identifies the class associated with each pixel in the map.

In some cases, the first neural network may be a semantic segmentation neural network. A semantic segmentation neural network receives an image and generates a classification of each pixel in the image. When the first neural network is a semantic segmentation neural network, the semantic segmentation neural network may be configured to classify each pixel as background/irrelevant or one of the plurality of anatomical structures of interest. The output is a segmentation map that identifies the class associated with each pixel. For example, if the anatomical structures of interest are the kidney and liver then a pixel may be assigned a "0" if it is neither the kidney or liver, a "1" if it is the kidney and a "2" if it is the liver.

In other cases, the first neural network may be an instance segmentation neural network. An instance segmentation neural network receives an image and outputs bounding boxes of objects identified therein, a classification of each identified object along and a pixel-wise map for each object that identifies the pixels in that bounding box that belong to that specific object. When the first neural network is an instance segmentation neural network, the instance segmentation neural network may be configured to identify the plurality of anatomical structures of interest.

In other cases, the first neural network may be a panoptic segmentation neural network. A panoptic segmentation neural network combines semantic segmentation and instance segmentation and produces a complete, non-overlapping pixel-wise labelling of an image that distinguishes both object classes and object instances. The output of a panoptic segmentation neural network is a single, unified segmentation of the entire image, in which every pixel is assigned a class label and an instance identifier, where applicable. The class identifier information may be stored in a separate map from the instance information. For example, if a panoptic segmentation model is configured to identify cars and trucks, if it identifies two cars and two trucks, then the pixels corresponding to each of the cars will be assigned the cars class/label, but pixels that correspond to different cars will be assigned different instance labels. When the first neural network is a panoptic segmentation model, the panoptic segmentation model may be configured to classify each pixel as background/irrelevant or one of the plurality of anatomical structures of interest. The output is a segmentation map that identifies the class associated with each pixel and an instance map. For example, if the anatomical structures of interest are the kidney and liver then a pixel may be assigned a 0 if it is neither the kidney or liver, a 1 if it is the kidney and a 2 if it is the liver.

**Pathological Condition Detection Module and Second Neural Network**

As described above, the pathological condition detection module 110 is configured to, in response to the ultrasound image verification module 108 determining that the set of one or more ultrasound image 104 is suitable for detecting the pathological condition, automatically determine from the set of one or more ultrasound image, whether the pathological condition is present. The pathological condition detection module 110 may be configured to determine whether the pathological condition is present by processing the set of one or more ultrasound image 104 using a second neural network 118 to generate a prediction of the presence of the pathological condition and/or a quantitative indication of the severity of the pathological condition. In some cases, the pathological condition may be free fluid in the anatomical region of interest.

*Prediction of the Presence of the Pathological Condition*

In some cases, the pathological condition detection module 110 is configured to process the set of one or more ultrasound image 104 using the second neural network 118 to generate a prediction of the presence of the pathological condition. In such cases, the second neural network 118 may be a classifier neural network. In some cases, the classifier neural network may be a single class classifier neural network that is configured to receive one or more ultrasound images and generate a single value that indicates whether the pathological condition is predicted to be present based on the one or more ultrasound images. In some cases, sigmoid aviation may be applied to the one value. In some cases, a classification threshold c may be applied to the value (e.g., after sigmoid activation) to binarize the prediction. The single class classifier neural network may be configured to receive and process one or more B-mode images or one or more M-mode images. Such a single-class classifier neural network may be trained, from sets of one or more ultrasound images of a particular mode with annotations indicating whether the pathological condition is present or not, using a loss function that penalizes incorrect binary classification predictions, such as, but not limited to, binary cross-entropy loss. The annotation may, for example, be of the form $y \in \{0,1\}$ where $y = 0$ indicates that the pathological condition is not present, and $y = 1$ indicates that the pathological condition is present. The annotations may be per image or per set of images depending on whether the classifier neural network is configured to process a single image at a time or a plurality of images at a time.

In other cases, the classifier neural network may be a multi-class classifier neural network that is configured to receive one or more ultrasound images and generate a plurality of values that indicate varying degrees of detection of the pathological condition For example, the multi-class classifier network may be configured to classify the set of one or more ultrasound image as (1) the pathological condition is definitely present, (2) the pathological condition is possibly present, or (3) the pathological condition is not present. This is an example only, and in other examples the multi-class classifier neural network may be configured to classify the set of one or more ultrasound image into more than three classes. The multi-class classifier neural network may be configured to receive and process one or more B-mode images or one or more M-mode images.

In some cases, each of the plurality values generated by the multi-class classifier neural network may correspond to one class. In such cases, SoftMax activation may be applied to each of the values, and the class with the highest value may be determined to be the predicted class. In such cases, the multi-class classification neural network may be trained from sets of one or more ultrasound images of a particular mode with multi-class annotations, using a loss function that penalized incorrect multi-class classification prediction, such as, but not limited to, cross-entropy.

In some cases, the number values in the plurality of values generated by the multi-class classifier neural network may be less than the number of classes detected by the multi-class classifier neural network. In such cases, the plurality of values may form a binary vector which progresses from the zero vector to the all ones vector. For example, [0,0] may indicate the pathological condition is not present, [0,1] may indicate that the pathological condition is possibly present, and [1,1] may indicate the pathological condition is definitely present. In these cases, sigmoid activation may be applied to each of the values. In such cases, the multi-class classification neural network may be trained from sets of one or more ultrasound images of a particular mode with multi-class vector annotations, using a loss function, such as, but not limited to, binary cross-entropy.

The classifier neural network (single-class or multi-class) may be configured to receive and process a single ultrasound image (B-mode image or M-mode image) at a time so as to generate a prediction for a single ultrasound image at a time; or receive and process a plurality of ultrasound images (a plurality of B-mode images from the same video, or a plurality of related M-mode images) as a whole so as to generate a prediction for the plurality of ultrasound images as a whole. Where the set of one or more ultrasound image 104 comprises a plurality of ultrasound images and the classifier neural network (single class or multi-class) is configured to receive and process a single ultrasound image at a time, the pathological condition detection module 110 may be configured to process each ultrasound image of the plurality of images using the classifier neural network to generate a prediction for that image and then generate a prediction for the plurality of images from the individual image predictions. In some cases, where the set of one or more ultrasound image comprises a plurality of M-mode images generated from different transducers that are physically spaced form each other by some distance threshold, and the second neural network is configured to process one M-image at a time, the pathological condition detection module 110 may determine that that the pathological condition is present if the pathological condition is predicted for any of the plurality of M-mode images.

*Quantitative Indication of the Severity of the Pathological Condition*

In some cases, the pathological condition detection module 110 is configured to process the set of one or more ultrasound image 104 using the second neural network to generate a quantitative indication of the severity of the pathological condition. Where the pathological condition is free fluid then the quantitative indication of the severity of the pathological condition may be an indication of the amount of free fluid detected.

In some cases, the second neural network may comprise a deep neural network that is configured to receive an ultrasound image and perform semantic segmentation thereon to generate a segmentation mask that indicates which pixels of the ultrasound image correspond to the pathological condition. Semantic segmentation is a computer vision task in which the goal is to categorize each pixel in an image into a class or object. For example, where the input ultrasound image has a height of $H$ and a width of $W$ then the second neural network may be configured to generate a segmentation mask $Y \in \{0,1\}^{H \times W}$ (i.e., the deep neural network is configured to perform $R^{H \times W} \rightarrow [0,1]^{H \times W}$). In the examples described herein, pixels that correspond to the pathological condition are set to one, and pixels that do not correspond to the pathological condition are set to zero. However, in other examples, pixels that correspond to the pathological condition are set to zero, and pixels that do not correspond to the pathological condition are set to one. In some cases, each element of the mask may be binarized by applying a threshold $c_s \in [0,1]$ thereto. In some cases, to reduce false-positive pixel-wise predictions, the pathological condition detection module 110 may be configured to set of pixels that are not within the largest $v \in N$ connected components of the segmentation mask generated by the deep neural network to a value (e.g., 0) indicating that the pixel does not correspond to the pathological condition. For example, the pathological condition detection module 110 may be configured to, for any pixel in a predicted mask that is identified as corresponding to the pathological condition (e.g., has a value of 1) that is not part of the first v contiguous regions (when the contiguous regions are ordered from largest to smallest by area), set the pixel value to indicate that it does not correspond to the pathological condition (e.g., set the pixel value to 0). In some cases, fully convolution semantic segmentation architectures, such as, but not limited to U-Net, may be used to train the deep neural network to generate a segmentation mask for an ultrasound image using ultrasound images annotated with a segmentation mask.

In other cases, the second neural network may comprise an object detection neural network that is configured to receive an ultrasound image and identify bounding boxes of continuous segments that correspond to the pathological condition (e.g., contain free fluid). The object detection neural network may be configured to implement any suitable object detection method, such as, but not limited to, YOLO, SSD and Fast R-CNN (region-based convolutional neural network). The object detection neural network may be trained using ultrasound images that have been annotated with bounding box labels for continuous segments that correspond to the pathological condition. Once the second neural network has identified a set of one or more bounding boxes that comprise continuous segments that correspond to the pathological condition, the pathological condition detection module 110 may generate a segmentation mask for each identified bound box that indicates which pixels of that bounding box correspond to the pathological condition. In some cases, where the pathological condition is free fluid, since free fluid appears nearly black in an ultrasound image, the pathological condition detection module 110 may be configured to generate a segmentation mask for an identified bounding box by indicating in the segmentation mask that each pixel that has an intensity less than a threshold $T$ does not correspond to the pathological condition. In other cases, the pathological condition detection module 110 may be configured to use other thresholding methods, such as, but not limited to, the thresholding method described in N. Otsu et al, "A threshold section method from gray-level histograms", Automatica, vol. 11, no. 285-296, pp. 22-27, 1975, which is herein incorporated by reference in its entirety. In other cases, the second neural network may be a neural network that is configured to both predict regions of interest and estimate segmentation masks within them (e.g. an instance segmentation neural network). Such a neural network may be implemented by, for example, a Mask R-CNN neural network.

Where a segmentation mask is created for each identified bounding box in an ultrasound image then a segmentation mask for the ultrasound image as a whole may be generated by combining the segmentation masks for the bounding boxes.

In some cases, where the ultrasound probe is a phased array probe or a curvilinear probe, then the pathological condition detection module 110 may be configured to confine the segmentation mask generated by the second neural network to the pixels of the ultrasound image that correspond to the ultrasound image's beam. In some cases, the pathological condition detection module 110 may be configured to confine the segmentation mask(s) generated by the second neural network to the pixels of the ultrasound image that correspond to the ultrasound image's beam by applying the image's beam mask, which may be computed as set out in the Applicant's US Patent Application No. 18/935,011, to the segmentation mask generated by the second neural network. For example, the predicted segmentation mask Y may be computed as the element-wise product $\hat{Y} \odot U$ where $Y$ is the segmentation mask computed by the second neural network and $U \in \{0,1\}^{H \times W}$ is the beam mask.

When the second neural network is used by the pathological condition detection module 110 to generate one or more segmentation masks for an ultrasound image, the pathological condition detection module 110 may be configured to determine a physical area of the ultrasound beam in the ultrasound image corresponding to the pathological condition based on the one or more segmentation masks. In one example, the physical area of the ultrasound beam in the ultrasound image corresponding to the pathological condition may be computed from the segmentation mask Y in accordance with equation (1):

$$A = R_y \sum_{Y[j,k]=1} R_x(j,k) \qquad (1)$$

where the constant $R_y$ is the axial resolution of the ultrasound image (in cm), and $R_x(j,k)$ is a function indicating the lateral scale of the ultrasound beam at pixel $[j,k]$ of the ultrasound image. For linear ultrasound probes, $R_x(j,k)$ is constant. For phased array or curved linear ultrasound probes with a depth $d$ (in cm), and r being the length of a line segment $\overline{p_0 p_3}$ in pixels, the lateral scale may be calculated in accordance with equation (2).

$$s(j,k) = \frac{2d \sqrt{((i - y_0)^2 + (j - x_0)^2)} \, \tan(\theta_0 / 2)}{rW} \qquad (2)$$

In some cases, instead of using the second neural network 118 to generate one or more segmentation masks, the second neural network may comprise a feature extractor neural network that is configured to receive one or more ultrasound images and output a value that represents the predicted severity of the pathological condition. Where the pathological condition is free fluid, the single value may be a real-valued prediction for the amount of fluid, measured in an appropriate unit of scale (e.g., $cm^2$). In some cases, the feature extractor neural network may be a convolutional neural network or a vision transformer. The feature extractor neural network may be trained using an annotated set of ultrasound images and a regression loss function that penalizes the distance between predictions and annotations (e.g., mean square error, mean absolute error). To generate the annotated ultrasound images, one may start with an ultrasound image annotated with a segmentation mask, then the amount of free fluid may be computed in accordance with, for example, equations (1) and (2) and annotated with the computed amount of free fluid.

*Alternative Second Neural Network*

In the examples described above, the second neural network is configured to receive and process only the set of one or more ultrasound image 104. However, in other examples, the second neural network may be configured to receive and process, in addition to the set of one or more ultrasound image, anatomical data generated from the output of the first neural network (e.g., the prediction of the presence and/or location of each of the plurality of anatomical structures); or a combination of the set of one or more ultrasound image 104 and the anatomical data generated from the output of the first neural network. Testing has shown that if the second neural network knows the location of anatomical structures surrounding the area where the pathological condition may occur, the second neural network is better able to localize the area and provide more correct boundaries therefore. For example, where the pathological condition is free fluid, testing has shown that if the second neural network knows the location of the liver and kidney, the second neural network is better able to localize fluid and provide more correct boundaries for the fluid areas.

In some cases, the anatomical data generated from the output of the first neural network may be an integer-value map for each ultrasound image of the set of one or more ultrasound image of the torso that comprises an integer for each pixel in the ultrasound image that identifies which anatomical structure, if any, the pixel corresponds to. For example, where the anatomical structures of interest include the liver and kidney, then a pixel may be assigned a "0" if it does not correspond to the liver or kidney, a "1" if it corresponds to the liver, and a "2" if it corresponds to the kidney. In some cases, as described above, the anatomical data may be generated by the first neural network itself. For example, where the first neural network is a sematic segmentation neural network, the first neural network may generate such an integer-value map. In other cases, the anatomical data may be generated from the output of the first neural network. For example, if the first neural network is an object detection neural network that outputs bounding boxes for identified objects in an image along with a classification of each identified object, then an integer-value map for the image may be generated therefrom.

In some cases, a binary mask (also referred to as a one-hot tensor) may be generated for each of the anatomical structures of interest from the integer-value map, where the mask for an anatomical structure indicates which pixels of the corresponding ultrasound image correspond to that anatomical structure. For example, if the anatomical structures comprise the liver and kidney, a mask may be generated for the liver that identifies the pixels of the ultrasound image that correspond to the liver, and a mask may be generated for the kidney that identifies the pixels of the ultrasound image that correspond to the kidney. Each mask has the same dimensions as the original image and has a value for each pixel that indicates whether the pixel is related to that anatomical structure or not.

In some cases, the second neural network may be configured to receive both the set of ultrasound images of the torso and a set of corresponding integer-value maps therefore. In such cases, each input may be sent to its own encoder (e.g., the backbone of a Mobile NetV3) within the second neural network and concatenated in feature space before being sent to region-of-interest prediction blocks as described above.

In other cases, the second neural network may be configured to receive as input, for each ultrasound image of the set of one or more ultrasound image, a combination of the original ultrasound image and the corresponding binary masks. Specifically, in some cases, the individual anatomical structure masks generated from the integer-value map may be superimposed on the original image. In particular, pixels that correspond to the anatomical structures may be coloured according to a colour map For example, the pixels in the original image that correspond to one anatomical structure (e.g., kidney) may be set to a certain colour (e.g., green), and pixels in the original image that correspond to another anatomical structure (e.g., liver) may be set to another colour (e.g., yellow).

In yet other cases, the second neural network may be configured to receive as input, for each ultrasound image, a tensor formed by the concatenation of the original ultrasound image and the integer-value mask. In other words, in these cases, for each ultrasound image the second neural network receives a tensor that comprises a first channel that includes the original image and a second channel that includes the integer-value mask. So, for an ultrasound image with width $w$ and height $h$, the second neural network may receive a tensor with shape $(h, w, 2)$. In other cases, instead of concatenating the original ultrasound image and the integer-value mask, the original ultrasound image may be concatenated with the anatomical structure masks. In other words, for each ultrasound image the second neural network receives a tensor that comprises a first channel that includes the original image, a second channel that comprises the mask for a first anatomical structure, a third channel that comprises the mask for a second anatomical structure and so on. Accordingly, for an ultrasound image with width $w$ and height $h$, the second neural network may receive a tensor with shape $(h, w, s+1)$ where $s$ is the number of anatomical structures.

**Additional Assessments**

In some cases, the assessment module 102 may be configured to perform additional tests or assessments (i) before the set of one or more ultrasound image is obtained by an ultrasound operator or (ii) based on data obtained while the set of one or more ultrasound image was being obtained, to determine whether the set of one or more ultrasound image is suitable for detection of the pathological condition.

Specifically, in some cases, prior to obtaining the set of one or more ultrasound images (e.g., a set of one or more B-modes images or a set of one or more M-modes images), the ultrasound technician may be configured to first obtain an A-mode ultrasound signal of the anatomical region of interest and the assessment module 102 may be configured to analyze the A-mode signal to quickly determine if the ultrasound probe is suitably situated to obtain ultrasound images of the anatomical region of interest. In particular, the assessment module 102 may be configured to analyze the A-mode signal to determine if the ultrasound probe needs to be moved or translated due to the presence of a bony obstacle. For example, when the ultrasound operator is attempting to obtain a subxiphoid view, it is desirable that the ultrasound operator position the ultrasound transducer so that it is not over the sternum.

In some cases, the assessment module 102 may be configured to analyze the A-mode ultrasound signal to determine whether the ultrasound probe is likely placed over an obstructing structure (e.g., bony structure) that may impede interpretation of any ultrasound image obtained from that ultrasound probe position. In some cases, the assessment module 102 may be configured to determine that an ultrasound probe is likely placed over an obstructing structure if, for an A-mode signal $a$, $\sum_i a_i < T_{bone}$, where $T_{bone}$ is an intensity threshold. In some cases, $T_{bone}$ may be empirically determined with a calibration set to determine an optimal value that separates A-mode signals where the ultrasound probe is positioned over an obstructing structure (e.g., bony structure) and A-mode signals where the ultrasound probe is not positioned over an obstructing structure (e.g., bony structure). Methods which may be used to identify $T_{bone}$ include, but are not limited to: maximum likelihood analysis, k-means with a threshold defined as the midpoint between the cluster's centroid, and the thresholding method set out in Otsu et al. Quickly determining from a simple ultrasound scan (i.e., A-mode signal) whether the ultrasound probe is likely placed over an obstructing structure (e.g., bony structure) can save the ultrasound operator from collecting M-mode images over obstructed locations and notify the ultrasound operator that it is desirable to move the ultrasound probe.

In some cases, additional tests or assessments may be performed by the assessment module 102 based on data obtained while the set of one or more ultrasound image was obtained, to determine whether the obtained set of one or more ultrasound image is suitable for detection of the pathological condition. In such cases, if it is determined by one or more of the additional tests or assessments that the obtained set of one or more ultrasound image is not suitable for detection of the pathological condition then the set of one or more ultrasound image may not be provided to the ultrasound image verification module 108 and the pathological condition detection module 110 for further processing.

In some cases, a suitable set of one or more ultrasound image for detecting the pathological condition may be obtained by holding the ultrasound transducer(s) in the right location and orientation with an appropriate pressure. In some cases, the ultrasound probe used to capture the set of one or more ultrasound image may comprise one or more pressure sensors located proximally to the transducers that are configured to measure the pressure that the ultrasound probe (i.e., the device comprising the one or more ultrasound transducer) applies to the patient. In such cases, the assessment module 102 may be configured to determine, from the output of the one or more pressure sensors while the set of one or more ultrasound image was being captured, whether proper pressure was applied by the ultrasound operator. In some cases, the assessment module 102 may be configured to determine whether proper pressure was applied by the ultrasound operator by comparing the output of the one or more pressure sensor to a predetermined pressure threshold. If it is determined that proper pressure was not applied by the ultrasound operator throughout the capture of the one or more ultrasound image, then it may be determined that the set of one or more ultrasound image is not suitable for detecting the pathological condition.

In some cases, the ultrasound probe used to capture the set of one or more ultrasound image may comprise an accelerometer located proximally to the transducer(s) that measures how quickly the transducer(s) are being moved. In such cases, the assessment module 102 may be configured to determine, from the output of the accelerometer while the set of one or more ultrasound image was being captured, whether the transducer(s) were being moved too quickly at any point during the capture of the set of one or more ultrasound image. In some cases, the assessment module 102 may be configured to determine whether the transducer(s) were being moved too quickly by comparing the output of the accelerometer to a predetermined acceleration threshold. In some cases, the acceleration threshold (which may represent the maximum acceptable acceleration) may be determined empirically by clinical experts. In some cases, the acceleration threshold may vary based on a plurality of conditions, such as, but not limited to, the particular anatomical region of interest (e.g., based on which view of the FAST examination is being obtained).

In some cases, the ultrasound probe used to capture the set of one or more ultrasound image may comprise one or more gyroscopes located proximally to the transducer(s) that measure the rotational movement of the transducer(s). In such cases, the assessment module 102 may be configured to determine, from the output of the one or more gyroscopes while the set of one or more ultrasound was being captured, whether the transducer(s) were rotated too quickly at any point during the capture of the set of one or more ultrasound image. In some cases, the assessment module 102 may be configured to determine whether the transducer(s) were rotated too quickly by comparing the output of the one or more gyroscopes to a predetermined rotational speed threshold. The rotational speed threshold (which may represent the maximum acceptable rotation speed) may be determined empirically by clinical experts. In some cases, the rotational speed threshold may vary based on a plurality of conditions, such as, but not limited the particular anatomical region of interest (e.g., based on which view of the FAST examination is being obtained).

## Second Example System

Reference is now made to FIG. 6 which illustrates a second example computing system 600 for detecting a pathological condition (e.g., free fluid) in an anatomical region of interest (e.g., a region of the torso) from a set of one or more ultrasound image of the anatomical region of interest. In this example, there is a single neural network configured and trained to perform anatomy detection and pathological condition detection (i.e., the anatomic detection and pathological condition detection may be performed concurrently).

The example computing system 600 comprises an assessment module 602 that is configured to receive a set of one or more ultrasound image 604 of an anatomical region of interest (e.g., a region of the torso) and (1) automatically determine if the set of one or more ultrasound image 604 is suitable for detecting the pathological condition and automatically determine from the set of one or more ultrasound image 604 if the pathological condition is present; and (2) if the set of one or more ultrasound image 604 is deemed suitable for detecting the pathological condition, output the determination of whether the pathological condition is present.

In some cases, one or more components of the computing system 600 may be implemented by one or more computers within the computing system 600, such as, but not limited to, computer 200 described above with respect to FIG. 2.

The comments above with respect to the set of one or more ultrasound images 104 of FIG. 1 equally apply to the set of one or more ultrasound image 604 of FIG. 6.

In some cases, the set of one or more ultrasound image 604 may be received, e.g., from an ultrasound machine, or a set of one or more ultrasound transducer, via a data ingestor 606. The data ingestor 606 may be configured to actively retrieve the set of one or more ultrasound image 604 or the data ingestor 606 may be configured to passively receive the set of one or more ultrasound image 604. In some cases, the set of one or more ultrasound image 604 received by the data ingestor 606 may be processed directly by the assessment module 602. In other cases, the data ingestor 606 may be configured to store the received set of one or more ultrasound image 604 in a repository 607 and the assessment module 602 may be configured to retrieve the set of one or more ultrasound image 604 from the repository 607. The repository 607 may be any mechanism or device, such as, but not limited to, memory, that can be used to store digital information.

The assessment module 602 is configured to receive a set of one or more ultrasound image 604 of an anatomical region of interest (e.g., a region of the torso) and (1) automatically determine if the set of one or more ultrasound image 604 is suitable for detecting the pathological condition and automatically determine from the set of one or more ultrasound image 604 if the pathological condition is present; and (2) if the set of one or more ultrasound image 604 is deemed suitable for detecting the pathological condition, output the determination of whether the pathological condition is present.

In the example shown in FIG. 6, the assessment module 602 comprises a combined neural network 609, an ultrasound image verification module 608, and a pathological condition detection module 610.

The combined neural network 609 is configured to process the set of one or more ultrasound image 604 to (i) detect a plurality of anatomical structures therein (which may be referred to as the anatomy detection 611) and (ii) detect the pathological condition (which may be referred to as the pathological detection 613).

In some cases, the anatomy detection 611 may comprise a prediction of the presence of each of the plurality of anatomical structures in the set of one or more ultrasound image 604 (e.g., a prediction of whether each of the plurality of anatomical structures is in the set of one or more ultrasound image 604). In other cases, the anatomy detection 611 may alternatively, or additionally, comprise a prediction of the location of each of the plurality of anatomical structures in the set of one or more ultrasound image 604.

In some cases, the pathological detection 613 may comprise a prediction of the presence of the pathological condition. In other cases, the pathological detection 613 may alternatively, or additionally, comprise a quantitative indication of the severity of the pathological condition. Where the pathological condition is free fluid then the quantitative indication of the severity of the pathological condition may be an indication of the amount of free fluid detected.

In some cases, where the anatomy detection 611 comprises a prediction of the presence of each of the plurality of anatomical features and the pathological detection 613 comprises a prediction of the presence of the pathological condition, the combined neural network 609 may comprise a classifier neural network. Such a classifier neural network may combine the features of the classifier neural network described above as implementing the first neural network and the classifier neural network described above as implementing the second neural network. Specifically, the classes which the classifier neural network identifies may comprise a class for each of the anatomical structures and at least one class that relates to the pathological condition. In some cases, there may be a single class related to the pathological condition and the value related to that class is intended to indicate whether the pathological condition is predicted to be present. In other cases, there may be multiple classes related to the pathological condition. For example, there may be a class that indicates that the pathological condition is present, a class that indicates that the pathological condition is possibly present, and a class that indicates that the pathological condition is not present. Example classes which may be used for anatomy detection and example classes which may be used for pathological condition detection and the configuration of the corresponding classifier neural network were described above with respect to the first and second neural network and equally apply to the combined neural network 609.

In some cases, where the anatomy detection 611 comprises a prediction of the position or location of each of the plurality of anatomical features and the pathological detection 613 comprises a prediction of the quantitative indication of the severity of the pathological condition of the pathological condition, the combined neural network 609 may comprise an object detection neural network. The object detection neural network outputs bounding boxes of identified objects and a classification of each identified object. In such cases, the classes of objects which may be detected may comprise a class for each of the plurality of anatomical structures and at least one class related to the pathological condition. In some cases, the class related to the pathological condition may be a class that comprises continuous segments that correspond to the pathological condition. In these cases, segmentation masks may be generated for the bounding boxes that comprise continuous segments that correspond to the pathological condition and an area related to the pathological condition can be computed therefrom as described above. Example classes which may be used for anatomy detection and example classes which may be used for pathological condition detection and the configuration of the corresponding object detection neural network were described above with respect to the first and second neural networks and equally apply to the combined neural network 609.

In other cases, where the anatomy detection 611 comprises a prediction of the position or location of each of the plurality of anatomical features and the pathological detection 613 comprises a prediction of the quantitative indication of the severity of the pathological condition of the pathological condition, the combined neural network 609 may comprise a segmentation neural network (e.g. a semantic segmentation neural network, an instance segmentation neural network, or a panoptic segmentation neural network). In these cases, the classes which the segmentation neural network segments the pixels of the images into include at least a class for each of the plurality of anatomical structures and at least one class for the pathological condition. The class for the pathological condition may indicate which pixels of the image relate to the pathological condition. As described above, the area related to the pathological condition can be determined from the pixels of the image that relate to the pathological condition.

The ultrasound image verification module 108 is configured to determine, from the anatomy detection 611, whether the set of one or more ultrasound image 604 is suitable for detecting the pathological condition by determining whether the set of one or more ultrasound image 604 adequately shows the anatomical region of interest. A set of one or more ultrasound image 604 may be deemed to adequately show the anatomical region of interest if the set of one or more ultrasound image 604 shows a combination of anatomical features associated with the anatomical region of interest

For example, if the anatomical region of interest is the RUQ view used in the FAST examination, then it may be determined that the set of one or more ultrasound image 604 is suitable for detecting a pathological condition if the liver and right kidney are present. Different anatomical regions of interest may be associated with different combinations of anatomical structures. For example, each of the four different views of the torso used in the FAST examination may be associated with a different combination of anatomical structures.

Any of the methods described above, with respect to the ultrasound image verification module 108 of FIG. 1, for determining, from the anatomy detection, whether a set of one or more ultrasound images are suitable for pathological condition detection may be implemented by the ultrasound image verification module 608 of FIG. 6.

In some cases, as shown in FIG. 6, the ultrasound image verification module 608 may be configured to output an indication of whether the set of one more ultrasound image 604 is suitable for detecting the pathological condition (which may be referred to as the suitable indication 614). In some cases, the suitable indication 614 may be output to a user, e.g., via a user interface 616 of the computing system 600.

The pathological condition detection module 610 is configured to receive an indication from the ultrasound image verification module 608 on whether the set of one or more ultrasound image 604 is suitable for pathological condition detection, and if the set of one or more ultrasound image 604 is suitable for pathological condition detection output a pathological condition indication 620, based on the pathological detection 613 output from the combined neural network 609. If the set of one or more ultrasound image is not suitable for pathological condition detection, then the pathological condition indication 620 is not output (e.g., the pathological detection 613 is ignored or discarded).

In some cases, the pathological condition detection module 610 may use the pathological detection 613 as the pathological condition indication 620. In other cases, the pathological condition detection module 610 may perform some processing on the pathological detection 613 to generate the pathological condition indication 620. For example, where the pathological detection 613 comprises one or more bounding boxes or one or more segmentation maps or masks, then the pathological condition detection module 610 may generate a quantitative indication (e.g., area) from the bounding boxes or segmentation maps or masks. In such cases, the pathological condition detection module 610 may be configured to compute the area in accordance with any of the methods described above. The pathological condition detection module 610 may perform any of the processing on the pathological detection 613 that the pathological condition detection module 110 of FIG. 1 performs on the output of the second neural network.

In some cases, as shown in FIG. 6, the pathological condition indication 620 may be provided to a user via a user interface 616 of the computing system 600. Specifically, the pathological condition indication 620 may be provided to a user device 624 that is connected over a data communication link 626 to the user interface 616. For example, the user may receive the pathological condition indication 620 via a web browser 628 or some other application that operates on the user device 624. In some cases, the ultrasound probe, the user device and/or the computing system 600 may be integrated into a single device.

In some cases, the combined neural network 609 may be configured to process one ultrasound image at a time - i.e., the combined neural network 609 may be configured to receive a single ultrasound image and generate anatomy detection information and pathological detection information for that image. In such cases, where the set of one or more ultrasound images comprise a plurality of ultrasound images, the ultrasound image verification module 608 and pathological condition detection module 610 may be configured to generate final anatomy detection information and final pathological detection information from the per image anatomy detection information and per image pathological detection information respectively. In other cases, the combined neural network 609 may be configured to process a plurality of ultrasound images as whole (e.g., as a video input) such that the combined neural network 609 generates single anatomy detection information and pathological detection information for the set of one or more ultrasound image.

Testing has shown that there are a number of benefits of using a single neural network to perform anatomy detection and pathological condition detection. Specifically, testing has shown that using a single neural network to perform anatomy detection and pathological condition detection can reduce the total inference time compared to having an anatomy detection neural network and a separate pathological condition detection neural network.

Furthermore, it has been discovered that for some pathological conditions, such as free fluid, the presence of the pathological condition may influence the appearance and form of relevant anatomical structures in the anatomical region of interest. Co-prediction of pathological free fluid and the surrounding anatomy can therefore be a useful tool that exploits these relationships for pattern recognition, while reducing the possibility of confusing free fluid with lookalike regions surrounding the examined view. For example, the medulla of the kidney can mimic free fluid, especially if the ultrasound probe isn't positioned correctly. Predicting the location of the kidney while simultaneously predicting free fluid would explicitly encourage the neural network to develop filters that recognize each individually based on patterns other than their texture.

Finally, testing has shown that training an object detection model to predict anatomical structures (e.g., kidney) in addition to instances of the pathological condition (e.g., free fluid) improves the object detection model's ability to localize the pathological condition (e.g., free fluid).

The assessment module 602 may be configured to perform any combination of the additional assessments described above with respect to the assessment module 102 of FIG. 1.

In some cases, the assessment module 602 may comprise a pre-processing module 622 which corresponds to the pre-processing module 122 of FIG. 1.

**Example Methods**

Reference is now made to FIG. 7 which illustrates an example method 700 for detecting a pathological condition (e.g., free fluid) in an anatomical region of interest (e.g., a region of the torso) from a set of one or more ultrasound image of the anatomical region of interest. The method 700 may be executed by one or more computers, such as the computer 200 of FIG. 2. The method 700 begins at block 702 where a set of one or more ultrasound image of an anatomical region of interest is received. In some examples, the anatomical region of interest may be one of the four views of the torso used in the FAST examination. The set of one or more ultrasound image may comprise a single ultrasound image or multiple ultrasound images. Each ultrasound image of the set of one or more ultrasound image may, for example, be an M-mode image or a B-mode image (e.g., a frame of a B-mode video). Once the set of one or more ultrasound image is received, the method 700 proceeds to block 704.

At block 704, the set of one or more ultrasound image received in block 702 is processed using one or more neural networks to (1) determine whether a plurality of anatomical structures are present in the set of one or more ultrasound image; and (2) determine whether the pathologic condition is present. If it is determined, from the anatomical structure determination, that the one or more ultrasound image is suitable for detecting the pathological condition then the determination of whether the pathological condition is present may be output; otherwise, an indication that the set of one or more ultrasound image is not suitable for detecting the pathological condition may be output. The method 700 may then end.

The one or more neural networks may be configured to determine whether a plurality of anatomical structures are present in the set of one or more ultrasound image by generating a prediction of a presence and/or position of each of a plurality of anatomical structures in the set of one or more ultrasound image.

The one or more neural networks may be configured to determine whether the pathological condition is present by generating a prediction of a presence of the pathological condition and/or a quantitative indication of a severity of the pathological condition.

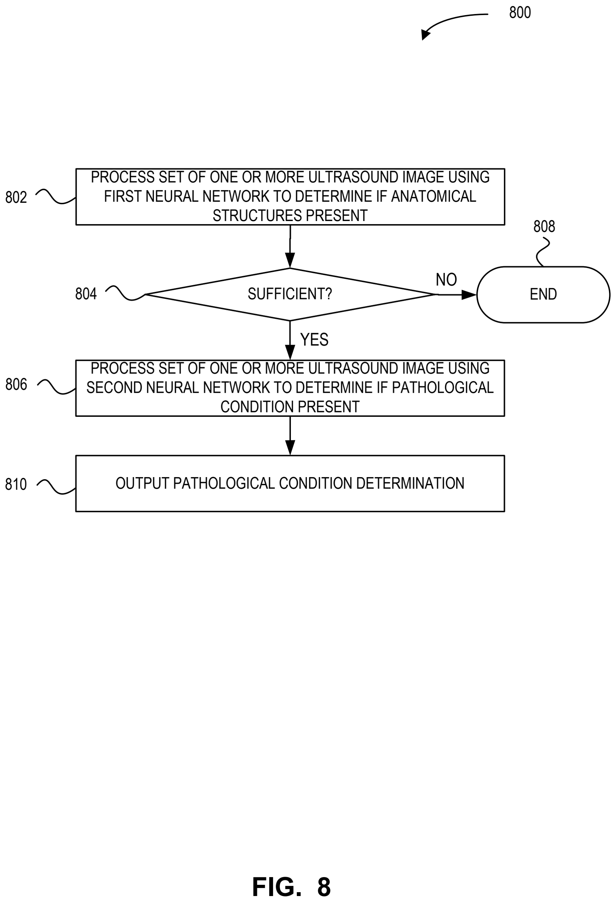

As described above, in some cases, the one or more neural network may comprise a first neural network that is configured and trained to perform the anatomical structure detection and a second, different neural network that is configured and trained to perform the pathological condition detection. An example method of implementing block 704 using such first and second neural networks is described below with respect to FIG. 8.

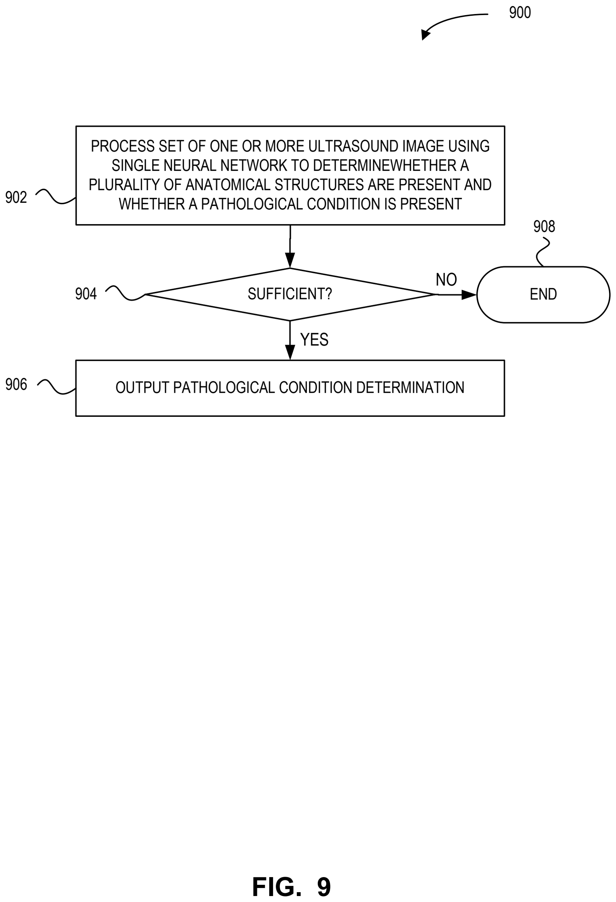

In other cases, the one or more neural network may comprise a single neural network configured and trained to perform both the anatomical structure detection and the pathological condition detection. An example method of implementing block 704 using such a single neural network is described below with respect to FIG. 9.

The set of one or more ultrasound image may relate to a patient, and in some cases, after the determination of whether the pathological condition is present has been output, the patient may be treated in accordance with the determination.

In some cases, the method 700 may also comprise, at block 712, pre-processing the set of one or more image prior to performing block 704. As described above, pre-processing the set of one or more images may comprise, for example, if the set of one or more image is in colour, converting the set of one or more image to greyscale; and/or removing extraneous information in the set of one or more images using, for example, a mask.

In some cases, the method 700 may also comprise obtaining training dataset of labelled sets of one or more ultrasound image for the one or more neural networks (block 714) and/or training the one or more neural networks using the training datasets (block 716). Example labels and annotations for training datasets for the first and second neural networks (which also can be used to train the combined neural network), and examples of how to train the first and second neural networks (and thus the combined neural network) based on the labelled training datasets were described above.

The method 700 of FIG. 7 may be repeated for different anatomical regions of interest. For example, at least blocks 702 and 704 (and optionally, the other blocks) may be repeated for a second set of one or more ultrasound image of the torso, wherein the set of one or more ultrasound image presents a first view of the torso and the second set of one or more ultrasound image presents a second, different, view of the torso. The one or more neural networks used to process the set of one or more ultrasound image associated with the first view of the torso may be different than the one or more neural networks used to process the set of one or more ultrasound image associated with the second view of the torso.

The method 700 of FIG. 7 may be used to implement the FAST examination by executing at least blocks 702 and 704 (and optionally, one or more other blocks) of the method 700 of FIG. 7 four times - once for a set of ultrasound images that present or represent the RUQ view, once for a set of ultrasound images that present or represent the LUQ view, once for a set of ultrasound images the present or represent the pelvic view, and once for a set of ultrasound images that present or represent the subxiphoid view. In some cases, the method 700 of FIG. 7 may not be executed for all views of the FAST examination. For example, in some cases, if the processing of a set of one or more ultrasound image associated with one view indicates internal bleeding, then the FAST examination may be deemed completed and the ultrasound images corresponding to the other views may not be obtained and/or processed.

Reference is now made to FIG. 8, which illustrates a first example method 800 for implementing block 704 of the method 700 of FIG. 7. In this example, the one or more neural network comprises a first neural network configured and trained to perform anatomy detection and a second, different neural network, configured to perform pathological detection. The method 800 begins at block 802, where the set of one or more ultrasound image is processed using a first neural network to generate a prediction of the presence and/or position of each of a plurality of anatomical structures in the set of one or more ultrasound image.

In some cases, the first neural network may be used to generate a prediction of the presence of each of the plurality of anatomical features in the set of one or more ultrasound image (e.g., a prediction of whether each of the plurality of anatomical features is in the set of one or more ultrasound image). In such cases, the first neural network may comprise a multi-label image classifier neural network, that is configured to receive one or more ultrasound images and generate a multi-element vector in which each element of the multi-element vector comprises the prediction of the presence of one of the plurality of anatomical structures in the received one or more ultrasound images. The multi-label classifier neural network may, for example, be a convolutional neural network or a vision transformer neural network.

In other cases, the first neural network may be used to generate a prediction of the location of each of a plurality of anatomical structures in the set of one or more ultrasound image. In such cases, the first neural network may comprise an object detection neural network.

In some cases, the first neural network may be configured to process one ultrasound image at a time - i.e., the first neural network may be configured to receive a single ultrasound image and generate a prediction as to the presence and/or location of the plurality of anatomical structures for that ultrasound image. In such cases, where the set of one or more ultrasound image comprises a plurality of ultrasound images each ultrasound image of the plurality of ultrasound images may be processed by the first neural network to generate a prediction of the presence and/or position of each of the plurality of anatomical structures in that ultrasound image, and a prediction of the presence and/or position of each of the plurality of structures for the set of one or more ultrasound image may be generated from the per image predictions (e.g. by combining the per image predictions). In other cases, the first neural network may be configured to process a plurality of ultrasound images as whole (e.g., as a video input) such that the first neural network can be used to generate a prediction for the set of one or more ultrasound image in one forward pass of the first neural network.

Once the prediction and/or location of the plurality of anatomical structures in the set of one or more ultrasound image has been generated, the method 800 proceeds to block 804.

At block 804, it is determined whether the set of one or more ultrasound image is suitable for detecting the pathological condition based on the predicted presence of the plurality of anatomical structures. If it is determined that the set of one or more ultrasound image is suitable for detecting the pathological condition, then the method 800 proceeds to block 806. If, however, it is determined that the set of one or more ultrasound image is not suitable for detecting the pathological condition, then the method 800 may end 808.

At block 806, in response to determining that the set of one or more ultrasound image is suitable for detecting the pathological condition, the set of one or more ultrasound image is processed using a second neural network to generate a prediction of presence of the pathological condition and/or a quantitative indication of a severity of the pathological condition.

In some cases, the second neural network is used to generate a prediction of the presence of the pathological condition. In such cases the second neural network may be a classifier neural network. In some cases, the classifier neural network may be a single class classifier neural network that is configured to receive one or more ultrasound images and generate a single value that indicates whether the pathological condition is predicted to be present based on the one or more ultrasound images. In other cases, the classifier neural network may be a multi-class classifier neural network that is configured to receive one or more ultrasound images and generate a plurality of values that indicate varying degrees of detection of the pathological condition For example, the multi-class classifier network may be configured to classify the set of one or more ultrasound image as (1) the pathological condition is present, (2) the pathological condition is possibly present, or (3) the pathological condition is not present.

In some cases, the second neural network is used to generate a quantitative indication of the severity of the pathological condition. Where the pathological condition is free fluid then the quantitative indication of the severity of the pathological condition may be an indication of the amount of free fluid detected. In some cases, the second neural network may comprise a semantic segmentation neural network that is configured to receive an ultrasound image and generate a segmentation mask that indicates which pixels of the ultrasound image correspond to the pathological condition. In other cases, the second neural network may comprise an object detection neural network that is configured to receive an ultrasound image and identify bounding boxes of continuous segments that correspond to the pathological condition (e.g., contain free fluid) and a model configured to generate a segmentation mask for each identified bound box that indicates which pixels of that bounding box correspond to the pathological condition. In either case, an area of the set of one or more ultrasound image corresponding to the pathological condition may be generated based on the one or more segmentation mask generated by the second neural network and the area may be used as the quantitative indication of the severity of the pathological condition.

In other cases, instead of generating one or more segmentation masks, the second neural network may comprise a feature extractor neural network that is configured to receive one or more ultrasound images and directly output a value that represents the predicted severity of the pathological condition.

Once the set of one or more ultrasound image has been processed by the second neural network, the method 800 proceeds to block 810 where the prediction of the presence of the pathological condition and/or a quantitative indication of a severity of the pathological condition and/or data based thereon is output.

Reference is now made to FIG. 9 which illustrates a second example method 900 for implementing block 704 of the method 700 of FIG. 7. In this example, the one or more neural network comprises a single neural network trained and configured to perform the anatomical structure detection and the pathological condition detection. The method 900 begins at block 902 where the one or more ultrasound image are processed using a single neural network to generate a prediction of a presence and/or position of each of a plurality of anatomical structures in the set of one or more ultrasound image of the torso, and a prediction of a presence of the pathological condition and/or a quantitative indication of a severity of the pathological condition. Example implementations of the single neural network were described above with respect to FIG. 6. Once the set of one or more ultrasound image has been processed by the single neural network, the method 900 proceeds to block 904.

At block 904, it is determined whether the set of one or more ultrasound image is suitable for detecting the pathological condition based on the predicted presence of the plurality of anatomical structures. If it is determined that the set of one or more ultrasound image is suitable for detecting the pathological condition, then the method 900 proceeds to block 906. If, however, it is determined that the set of one or more ultrasound image is not suitable for detecting the pathological condition, then the method 900 may end 908 or an indication that the set of one or more ultrasound image is not suitable for pathological detection may be output.

At block 906, in response to determining that the set of one or more ultrasound image is suitable for pathological detection, the prediction of a presence of the pathological condition and/or a quantitative indication of a severity of the pathological condition and/or data based thereon is output. The method 900 may then end.

Various systems or processes have been described to provide examples of embodiments of the claimed subject matter. No such example embodiment described limits any claim and any claim may cover processes or systems that differ from those described. The claims are not limited to systems or processes having all the features of any one system or process described above or to features common to multiple or all the systems or processes described above. It is possible that a system or process described above is not an embodiment of any exclusive right granted by issuance of this patent application. Any subject matter described above and for which an exclusive right is not granted by issuance of this patent application may be the subject matter of another protective instrument, for example, a continuing patent application, and the applicants, inventors or owners do not intend to abandon, disclaim or dedicate to the public any such subject matter by its disclosure in this document.

For simplicity and clarity of illustration, reference numerals may be repeated among the figures to indicate corresponding or analogous elements. In addition, numerous specific details are set forth to provide a thorough understanding of the subject matter described herein. However, it will be understood by those of ordinary skill in the art that the subject matter described herein may be practiced without these specific details. In other instances, well-known methods, procedures, and components have not been described in detail so as not to obscure the subject matter described herein.

The terms "coupled" or "coupling" as used herein can have several different meanings depending in the context in which these terms are used. For example, the terms coupled or coupling can have a mechanical, electrical, or communicative connotation. For example, as used herein, the terms coupled or coupling can indicate that two elements or devices are directly connected to one another or connected to one another through one or more intermediate elements or devices via an electrical element, electrical signal, or a mechanical element depending on the particular context. Furthermore, the term "operatively coupled" may be used to indicate that an element or device can electrically, optically, or wirelessly send data to another element or device as well as receive data from another element or device.

As used herein, the wording "and/or" is intended to represent an inclusive- or. That is, "X and/or Y" is intended to mean X or Y or both, for example. As a further example, "X, Y, and/or Z" is intended to mean X or Y or Z or any combination thereof.

Terms of degree such as "substantially", "about", and "approximately" as used herein mean a reasonable amount of deviation of the modified term such that the result is not significantly changed. These terms of degree may also be construed as including a deviation of the modified term if this deviation would not negate the meaning of the term it modifies.

Any recitation of numerical ranges by endpoints herein includes all numbers and fractions subsumed within that range (e.g., 1 to 5 includes 1, 1.5, 2, 2.75, 3, 3.90, 4, and 5). It is also to be understood that all numbers and fractions thereof are presumed to be modified by the term "about" which means a variation of up to a certain amount of the number to which reference is being made if the result is not significantly changed.

Some elements herein may be identified by a part number, which is composed of a base number followed by an alphabetical or subscript-numerical suffix (e.g., 112a, or 112b). All elements with a common base number may be referred to collectively or generically using the base number without a suffix (e.g., 112).

The systems and methods described herein may be implemented as a combination of hardware or software. In some cases, the systems and methods described herein may be implemented, at least in part, by using one or more computer programs, executing on one or more programmable devices including at least one processing element, and a data storage element (including volatile and non-volatile memory and/or storage elements). These systems may also have at least one input device (e.g., a pushbutton keyboard, mouse, a touchscreen, and the like), and at least one output device (e.g., a display screen, a printer, a wireless radio, and the like) depending on the nature of the device. Further, in some examples, one or more of the systems and methods described herein may be implemented in or as part of a distributed or cloud-based computing system having multiple computing components distributed across a computing network. For example, the distributed or cloud-based computing system may correspond to a private distributed or cloud-based computing cluster that is associated with an organization. Additionally, or alternatively, the distributed or cloud-based computing system be a publicly accessible, distributed or cloud-based computing cluster, such as a computing cluster maintained by Microsoft Azure™, Amazon Web Services™, Google Cloud™, or another third-party provider. In some instances, the distributed computing components of the distributed or cloud-based computing system may be configured to implement one or more parallelized, fault-tolerant distributed computing and analytical processes, such as processes provisioned by an Apache Spark™ distributed, cluster-computing framework or a Databricks™ analytical platform. Further, and in addition to the CPUs described herein, the distributed computing components may also include one or more graphics processing units (GPUs) capable of processing thousands of operations (e.g., vector operations) in a single clock cycle, and additionally, or alternatively, one or more tensor processing units (TPUs) capable of processing hundreds of thousands of operations (e.g., matrix operations) in a single clock cycle.

Some elements that are used to implement at least part of the systems, methods, and devices described herein may be implemented via software that is written in a high-level procedural language such as object-oriented programming language. Accordingly, the program code may be written in any suitable programming language such as Python or Java, for example. Alternatively, or in addition thereto, some of these elements implemented via software may be written in assembly language, machine language or firmware as needed. In either case, the language may be a compiled or interpreted language.

At least some of these software programs may be stored on a storage media (e.g., a computer readable medium such as, but not limited to, read-only memory, magnetic disk, optical disc) or a device that is readable by a general or special purpose programmable device. The software program code, when read by the programmable device, configures the programmable device to operate in a new, specific, and predefined manner to perform at least one of the methods described herein.

Furthermore, at least some of the programs associated with the systems and methods described herein may be capable of being distributed in a computer program product including a computer readable medium that bears computer usable instructions for one or more processors. The medium may be provided in various forms, including non-transitory forms such as, but not limited to, one or more diskettes, compact disks, tapes, chips, and magnetic and electronic storage. Alternatively, the medium may be transitory in nature such as, but not limited to, wire-line transmissions, satellite transmissions, internet transmissions (e.g., downloads), media, digital and analog signals, and the like. The computer usable instructions may also be in various formats, including compiled and non-compiled code.

While the above description provides examples of one or more processes or systems, it will be appreciated that other processes or systems may be within the scope of the accompanying claims.

To the extent any amendments, characterizations, or other assertions previously made (in this or in any related patent applications or patents, including any parent, sibling, or child) with respect to any art, prior or otherwise, could be construed as a disclaimer of any subject matter supported by the present disclosure of this application, Applicant hereby rescinds and retracts such disclaimer. Applicant also respectfully submits that any prior art previously considered in any related patent applications or patents, including any parent, sibling, or child, may need to be revisited.

## Claims

1.  A system to detect a pathological condition based on a set of one or more ultrasound image of a torso, the system comprising:

    a memory storing instructions; and
    at least one processor coupled to the memory, the at least one processor configured to execute the instructions to perform a method comprising:

      processing the set of one or more ultrasound image of the torso using one or more neural networks to generate:

a prediction of a presence and/or position of each of a plurality of anatomical structures in the set of one or more ultrasound image of the torso, and

a prediction of a presence of the pathological condition and/or a quantitative indication of a severity of the pathological condition; and

in response to determining, based on the prediction of the presence and/or position of the plurality of anatomical structures, that the set of one or more ultrasound image is suitable for detecting the pathological condition, outputting the prediction of the presence of the pathological condition and/or the quantitative indication of the severity of the pathological condition.

2. The system of claim 1, wherein the one or more neural networks comprises a single neural network configured to generate the prediction of the presence and/or position of each of the plurality of anatomical structures in the set of one or more ultrasound image of the torso, and the prediction of the presence of the pathological condition and/or the quantitative indication of the severity of the pathological condition.

3. The system of claim 1, wherein the one or more neural networks comprises a first neural network configured to generate the prediction of the presence and/or position of each of the plurality of anatomical structures in the set of one or more ultrasound image of the torso and a second, different neural network configured to generate the prediction of the presence of the pathological condition and/or the quantitative indication of the severity of the pathological condition.

4. The system of claim 3, wherein the method further comprises only processing the set of one or more ultrasound image of the torso using the second, different neural network in response to determining that the set of one or more ultrasound image of the torso is suitable for detecting the pathological condition.

5. The system of claim 3 or claim 4, wherein the second, different neural network is configured to process the set of one or more ultrasound image of the torso in conjunction with data generated from the prediction of the presence and/or position of each of the plurality of anatomical structures in the set of one or more ultrasound image of the torso.

6. The system of any preceding claim, wherein the one or more neural networks comprises a classifier neural network configured to generate the prediction of the presence of each of the plurality of anatomical structures in the set of one or more ultrasound image of the torso and the prediction of the presence of each of the plurality of anatomical structures comprises a multi-element vector that comprises an element for each of the plurality of anatomical structures that indicates the prediction of the presence of that anatomical structure in the set of one or more ultrasound image of the torso.

7. The system of any of claims 1 to 5, wherein the one or more neural networks comprises an object detection neural network configured to generate the prediction of the position of each of the plurality of anatomical structures in the set of one or more ultrasound image of the torso.

8. The system of any of claims 1 to 5, wherein the one or more neural networks comprises a segmentation neural network configured to generate the prediction of the position of each of the plurality of anatomical structures in the set of one or more ultrasound image of the torso.

9. The system of any preceding claim, wherein determining, based on the prediction of the presence and/or position of the plurality of anatomical structures, whether the set of one or more ultrasound image of the torso is suitable for detecting the pathological condition comprises:

generating a binary decision as to whether the set of one or more ultrasound image of the torso is suitable for detecting the pathological condition; or

generating a ternary decision as to whether the set of one or more ultrasound image of the torso is suitable for detecting the pathological condition, the ternary decision indicating whether the set of one or more ultrasound image of the torso is (i) suitable for detecting the pathological condition, (ii) not suitable for detecting the pathological condition, or (iii) comprises an anatomically impossible combination of the plurality of anatomical structures.

10. The system of any preceding claim, wherein the set of one or more ultrasound image of the torso comprises a plurality of ultrasound images of the torso and processing the set of one or more ultrasound image of the torso using the one or more neural networks to generate the prediction of the presence and/or position of each of the plurality of anatomical structures in the set of one or more ultrasound image comprises:

processing the plurality of ultrasound images of the torso as a whole using a neural network of the one or more neural networks to generate the prediction of the presence and/or position of each of the plurality of anatomical structures; or

processing each ultrasound image of the plurality of ultrasound images of the torso using a neural network of the one or more neural networks to generate a prediction of the presence and/or position of each of the plurality of anatomical structures in that ultrasound image, and generating the prediction of the presence and/or position of each of the plurality of anatomical structures in the set of one or more ultrasound image of the torso based on the prediction of the presence and/or position of that anatomical structure for each ultrasound image of the plurality of ultrasound images of the torso.

11. The system of any preceding claim, wherein the one or more neural networks comprises a classifier neural network configured to generate the prediction of the presence of the pathological condition, and the prediction of the presence of the pathological condition comprises (i) a single value that indicates whether the pathological condition is predicted to be present; or (ii) a plurality of values that indicate varying degrees of detection of the pathological condition.

12. The system of any of claims 1 to 10, wherein the one or more neural networks comprises a segmentation neural network configured to generate the quantitative indication of the severity of the pathological condition, the quantitative indication of the severity of the pathological condition comprising, for one or more ultrasound image in the set of one or more ultrasound image of the torso, a segmentation mask that indicates which pixels of the ultrasound image correspond to the pathological condition.

13. The system of any of claims 1 to 10, wherein the one or more neural networks comprises a feature extractor configured to generate the quantitative indication of the severity of the pathological condition, wherein the quantitative indication of the severity of the pathological condition comprises a value that represents a predicted severity of the pathological condition.

14. The system of any preceding claim, wherein the pathological condition is free fluid in the torso, and optionally, wherein the quantitative indication of the severity of the pathological condition is an estimate of a quantity of the free fluid in the torso.

15. A method for detecting a pathological condition based on a set of one or more ultrasound image of a torso, the method comprising, at one or more processors:

processing the set of one or more ultrasound image of the torso using one or more neural networks to generate:

a prediction of a presence and/or position of each of a plurality of anatomical structures in the set of one or more ultrasound image; and

a prediction of presence of the pathological condition and/or a quantitative indication of a severity of the pathological condition; and

in response to determining, based on the prediction of the presence and/or position of the plurality of anatomical structures, that the set of one or more ultrasound image of the torso is suitable for detecting the pathological condition, outputting the prediction of the presence of the pathological condition and/or the quantitative indication of the severity of the pathological condition.

**FIG. 1**

FIG. 2

FIG. 3A

FIG. 3B

FIG. 3C

(x_2, y_2)   (x_4, y_4)

θ_0   (x_0, y_0)   (x_1, y_1)   (x_3, y_3)

Phased array

**FIG. 4C**

(x_0, y_0)   θ_0   (x_2, y_2)   (x_4, y_4)

(x_1, y_1)   (x_3, y_3)

Curvilinear

**FIG. 4B**

(x_2, y_2)   (x_4, y_4)

(x_1, y_1)   (x_3, y_3)

Linear

**FIG. 4A**

**FIG. 5**

**FIG. 6**

EP 4 783 193 A1

700

| 714 | GENERATE LABELLED TRAINING DATASETS FOR NEURAL NETWORK(S) |

| 716 | TRAIN NEURAL NETWORK(S) USING LABELLED TRAINING DATASETS |

| 702 | RECEIVE A SET OF ONE OR MORE ULTRASOUND IMAGE |

| 712 | PRE-PROCESS SET OF ONE OR MORE ULTRASOUND IMAGE |

| 704 | PROCESS SET OF ONE OR MORE ULTRASOUND IMAGE USING ONE OR MORE NEURAL NETWORKS TO DETERMINE WHETHER A PLURALITY OF ANATOMICAL STRUCTURES ARE PRESENT AND WHETHER A PATHOLOGICAL CONDITION IS PRESENT; AND

OUTPUT THE PATHOLOGICAL CONDITON DETERMINATION IF IT IS DETERMINED, FROM THE ANATOMICAL STRUCTURE DETERMINATION, THAT THE SET OF ONE OR MORE ULTRASOUND IMAGE IS SUITABLE FOR PATHOLOGICAL DETECTION |

**FIG. 7**

800

802 — PROCESS SET OF ONE OR MORE ULTRASOUND IMAGE USING FIRST NEURAL NETWORK TO DETERMINE IF ANATOMICAL STRUCTURES PRESENT

808

804 — SUFFICIENT? — NO — END

YES

806 — PROCESS SET OF ONE OR MORE ULTRASOUND IMAGE USING SECOND NEURAL NETWORK TO DETERMINE IF PATHOLOGICAL CONDITION PRESENT

810 — OUTPUT PATHOLOGICAL CONDITION DETERMINATION

**FIG. 8**

900

902 — PROCESS SET OF ONE OR MORE ULTRASOUND IMAGE USING SINGLE NEURAL NETWORK TO DETERMINEWHETHER A PLURALITY OF ANATOMICAL STRUCTURES ARE PRESENT AND WHETHER A PATHOLOGICAL CONDITION IS PRESENT

908

904 — SUFFICIENT? — NO — END

YES

906 — OUTPUT PATHOLOGICAL CONDITION DETERMINATION

**FIG. 9**

Europäisches
Patentamt
European
Patent Office
Office européen
des brevets

# EUROPEAN SEARCH REPORT

**Application Number**

EP 26 15 3915

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/013114 A1 (KONINKLIJKE PHILIPS NV [NL]) 18 January 2024 (2024-01-18) * claims 1-20 * * paragraph [0002] - paragraph [0018] * * paragraph [0021] * * paragraph [0027] - paragraph [0030] * * paragraph [0039] - paragraph [0045] * * paragraph [0047] * * paragraph [0057] - paragraph [0068] * ----- | 1-15 | INV. G16H50/20 G06T7/00 G16H50/30 ADD. G16H30/40 |
| X | US 2024/212138 A1 (POTTER ILKAY YILDIZ [US] ET AL) 27 June 2024 (2024-06-27) * claims 1-20 * * paragraph [0007] * * paragraph [0013] - paragraph [0020] * * paragraph [0026] - paragraph [0035] * * paragraph [0040] - paragraph [0050] * ----- | 1-15 | |
| X | CA 2 845 044 A1 (JOINTVUE LLC [US]) 21 February 2013 (2013-02-21) * claims 11-15 * * paragraph [0001] * ----- | 1-15 | TECHNICAL FIELDS SEARCHED (IPC) G16H G06T |
| X | US 2022/386998 A1 (MEHANIAN COUROSH [US] ET AL) 8 December 2022 (2022-12-08) * paragraph [0019] - paragraph [0022] * * paragraph [0072] * * paragraph [0079] - paragraph [0087] * * paragraph [0093] * ----- | 1-15 | |

The present search report has been drawn up for all claims

| Place of search | Date of completion of the search | Examiner |
|---|---|---|
| Munich | 10 June 2026 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

  ...........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 26 15 3915

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2026

| Patent document cited in search report | | Publication date | Patent family member(s) | | Publication date |
|---|---|---|---|---|---|
| WO 2024013114 | A1 | 18-01-2024 | NONE | | |
| US 2024212138 | A1 | 27-06-2024 | NONE | | |
| CA 2845044 | A1 | 21-02-2013 | CA | 2845044 A1 | 21-02-2013 |
| | | | EP | 2741674 A1 | 18-06-2014 |
| | | | US | 2014163375 A1 | 12-06-2014 |
| | | | US | 2020113543 A1 | 16-04-2020 |
| | | | US | 2023210499 A1 | 06-07-2023 |
| | | | WO | 2013025613 A1 | 21-02-2013 |
| US 2022386998 | A1 | 08-12-2022 | CA | 3109818 A1 | 20-02-2020 |
| | | | CN | 113261066 A | 13-08-2021 |
| | | | EP | 3837695 A1 | 23-06-2021 |
| | | | US | 2020054306 A1 | 20-02-2020 |
| | | | US | 2022386998 A1 | 08-12-2022 |
| | | | WO | 2020037266 A1 | 20-02-2020 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 935011

**Non-patent literature cited in the description**

- **N. OTSU et al.** A threshold section method from gray-level histograms. *Automatica*, 1975, vol. 11 (285-296), 22-27